# EUROPEAN PATENT SPECIFICATION

(11) **EP 4 247 284 B1**
(45) Date of publication and mention of the grant of the patent: **20.08.2025**
(21) Application number: 21827337.3
(22) Date of filing: 22.11.2021
(51) Int. Cl.: A61B 18/14, A61B 18/00, A61B 18/12

(54) **BRAIDED OR WOVEN ELECTRODES**
GEFLOCHTENE ODER GEWEBTE ELEKTRODEN
ÉLECTRODES TRESSÉES OU TISSÉES

(30) Priority: 23.11.2020 US 202063117433 P
(43) Date of publication of application: 27.09.2023
(60) Divisional application: 25189975.3
(73) Proprietor: Pulse Biosciences, Inc., Hayward, CA 94545 (US)
(72) Inventor: TUROVSKIY, Roman, Hayward, CA 94545 (US); CONNOLLY, Richard J., Hayward, CA 94545 (US)
(74) Representative: IP Sextant s.r.l.
(86) International application number: PCT/US2021/060408
(87) International publication number: WO 2022/109431

(56) References cited:
- EP-B1- 1 210 023
- US-A1- 2012 209 262
- US-A1- 2020 008 870
- US-A1- 2020 261 720

## Description

### FIELD

Described herein are medical apparatuses (e.g., devices, systems, etc.) and methods, the methods not forming part of the invention, that may be used to perform medical operations to treat patients. Specifically, described herein are electrical applicators having one or more funnel-shaped electrodes, which may also include a center electrode.

### BACKGROUND

Short, high-field strength electric pulses have been described for electromanipulation of biological cells. For example, electric pulses may be used in treatment of animal (including human) tissue, such as tumors, including but not limited to basal cell carcinoma, squamous cell carcinoma, and melanoma. A variety of different applicators, e.g., electrodes, have been described for treating tissues, including needle electrodes and plate electrodes. For example, document US 2012/209262 A1 discloses an apparatus for mapping and/or ablating tissue including a braided conductive member. Document US 2020/008870 A1_discloses an ablation and implant system for ablating and occluding the left atrial appendage.

It would be particularly advantageous to be able to treat a patient tissue, including larger regions of tissue, with electrodes that may be manipulated within the body (e.g., minimally or non-invasively) or invasively, including treating larger surface areas. However, because of the high therapeutic voltages, as well as the very fast pulse times, applicators for delivery of such pulsed nanosecond energy devices must be configured so as to avoid damaging tissues or otherwise harming the patient. The risks of delivering high voltage energy, such risks including electrical shock, arcing, burns, internal-organ damage, and cardiac arrhythmias, are even more acute when the high voltage device is intended to be inserted into the body.

Thus, it would be beneficial to provide devices, such as applicators (electrode devices, electrical applicators, etc.), including elongate flexible applicators, that may apply high voltage, sub-microsecond electrical pulses to treat patients while mitigating the above-mentioned risks.

### SUMMARY OF THE DISCLOSURE

The invention provides an electrical applicator device according to claim 1. For better understanding of the invention the present disclosure describes also further apparatuses (including instruments and devices, such as elongate applicator tools, e.g., configured as applicator catheters, applicator scopes, tubes, etc.), and methods, said methods not forming part of the invention, for the treatment of a patient that may use them to more effectively apply therapeutic energy, including but not limited to short, high field strength electric pulses, while minimizing or avoiding the risk of harming non-target tissue. In some examples, these apparatuses may be used within a vessel (e.g., blood vessel) or other body lumen. These applicators may be referred to herein as electrode devices or electrical applicators, and may be used for minimally invasive procedures, and may be particularly well suited, for example, for treatments of various conditions, disorders and diseases, such as, but not limited to cancer (and other types of abnormal tissue growth), and the like. These applications may be also particularly well suited for use with various fully and partially automated systems, such as robotic systems. In particular, the apparatuses described herein may be configured as apparatuses (e.g., catheter apparatuses, catheter applicator tips, endoscopes, laparoscopes, etc.) that can be used with a variety of different generator systems, as will be described in greater detail herein.

In general, according to one aspect, the electrical applicators described herein include a braided or woven body that forms one or more funnel shapes having a distal-facing end (e.g., the opening of the funnel) that includes an electrically conductive region, e.g., an electrode. These funnel-shaped electrodes may include a second electrically conductive region (e.g., electrode) that may be surrounded by the outer, distal-facing edge of the funnel. In any of these examples, the second electrically conductive region may be in the same plane as the electrically conductive region(s) on the distal-facing end region of the funnel. In some examples, the second electrically conductive region, which may be positioned centrally relative to the funnel shape, may be coupled to an elongate member that allows the relative longitudinal position of the electrode to be adjusted relative to the distal-facing end region of the funnel.

In some examples the electrical applicator includes a second (or more) funnel shaped electrode concentrically within the outer funnel shape.

Any of these devices may be configured so that the distal end, including the funnel shape and second (e.g., central) electrode is steerable from a proximal end of the device. For example, the devices described herein may include a bending region proximal to the braided or woven body that may be controllably bent by one or more wires or tendons.

Any of these devices may be configured so that the funnel shape is formed prior to deployment (e.g., within the body or immediately before delivery to the body). Prior to deploying, the woven or braided material that will form the funnel shape may be a tube of material, which may be formed, at least in part, of an electrically conductive material. The region of the woven or braided material that will form distal-facing end region of the funnel may be un-insulated, while the other regions may be insulated (e.g., the region proximal and/or distal to the distal-facing opening of the funnel.

The funnel shape may be formed, for example, by coupling an inner deployment member (e.g., a puller, cannula, etc.) to a distal attachment site on the woven or braided material, which may initially be formed as a tubular body, referred to herein as a braided or woven body. The elongate deployment member may pass through the inner lumen of the braided or woven body. The proximal end of the braided or woven body may be coupled to another elongate member, such as a wire, cannula, tube, etc., or another portion of the device. Alternatively or additionally, the braided or woven material may be held within an outer cannula. The braided or woven body of the device may be deployed into the funnel shape by pulling the deployment member and/or by pushing the deployment member. For example, pulling the deployment member proximally and/or driving the member coupled to the proximal end of the braided or woven material may deploy the braided or woven material so the braided or woven material bends radially and inverts into itself (e.g., into the lumen of the tube of braided or woven material) to form a flared-out funnel shape. The distal-facing end (the large end) of the funnel may be configured so that all or a portion of this distal-facing end region is electrically exposed, e.g., forming the electrode. In some examples, the entire perimeter of the distal-facing end region is exposed, forming a circumferential electrode at or near the distal end. In some examples, multiple exposed (e.g. non-insulated) regions may be positioned around the perimeter of the funnel shape. Each non-insulated region may form a separate electrode, which may each be insulated (and separated) from each other. These electrodes may be separately addressable (e.g., coupled to an electrical contact at a proximal end of the device) or they may be electrically coupled together. When the device includes multiple, separately addressable electrodes, these electrodes may be insulated from each other.

For example described herein are electrical applicators that include: a braided or woven body having a distal end and a proximal end; an inner deployment member extending within the braided or woven body and coupled to a distal attachment site of the braided or woven body, wherein the inner deployment member is configured to be movable to invert a distal end region of the braided or woven body to form a funnel shape having a distal-facing outer region; a first electrically conductive region formed at least partially around the distal-facing outer region; and a second electrically conductive region, wherein the second electrically conductive region is configured to be surrounded by the distal-facing outer region and within the funnel shape when the inner deployment member is withdrawn proximally.

Any of these devices may include an elongate proximal body extending proximally from the funnel shape. This elongate body may be flexible or rigid or may be flexible over a portion of a length, e.g., near the distal end, and rigid over another portion of a length, e.g., near the proximal end. The elongate body may include, enclose and/or house one or more electrical conductors electrically connecting the electrically conductive contacts (electrodes) at the distal end region of the device to one or more electrical contacts at the proximal end region. These proximal electrical contracts may be configured to couple with a pulse generator for applying electrical energy from the device. In particular, these electrical contacts at the proximal end may be configured for coupling to a pulse generator configured to generate a plurality of electrical pulses having amplitude of at least 0.1 kV and a duration of less than 1000 nanoseconds. Thus, any of these devices may include a pair of electrical connectors at a proximal end region of the elongate proximal body in electrical communication with the first and second electrically conductive regions.

The devices described herein may include a braided or woven body that is formed, at least in part, of a conductive wire. The conductive wire may be, e.g., a stainless steel or a shape memory material (e.g., shape memory alloy such as a nickel-titanium alloy). The individual wires may be insulated, as described above. The first electrically conductive region may be formed of an uninsulated portion of the conductive wire forming the braided or woven body. In some variations, at least the portion of the braided or woven body proximal to the distal-facing outer region, when the funnel shape is deployed, is electrically insulated.

The second electrically conductive region may be positioned distal to the distal attachment site of the braided or woven body. When the device is deployed, to form the funnel shape, this second electrically conductive region may be positioned within (or at least partially within) the distal-facing opening of the funnel.

In some examples, the second electrically conductive region may be configured to be radially expanded when the inner deployment member is withdrawn proximally. For example, the second electrically conductive region may also be formed of the braided or woven material (or a second braided or woven material) that may be pulled proximally to flatten (or expand) radially forming a large contact for the second electrically conductive region.

In general, these devices may be configured as bipolar and/or monopolar devices and may be operated as either a bipolar electrical applicator or a monopolar electrical device.

The device of claim 1, further comprising a positioning member extending proximally from the second electrically conductive region within the braided or woven body and configured to allow the longitudinal position of the second electrically conductive region to be adjusted relative to the distal-facing outer region of the funnel shape.

As mentioned above the conductive region(s) on the outer distal-facing end region of the funnel may be configured as a single conductive region (e.g., electrode) or the conductive regions may include additional, separate conductive regions from electrodes (e.g., a third or more conductive regions) on the first distal-facing outer region. These separate conductive regions may be electrically insulated from each other.

Any of these devices may be configured to include or be operated with an outer member. The device may be deployed through an outer member, which may allow the user to control the outer diameter of the expanded funnel(s). For example, the braided or woven body may be slidably positioned within the outer member and configured to extend distally out of the outer member for deployment of the funnel shape. In some examples, this may allow the diameter of the funnel (and therefore the first electrically conductive region/electrode) to be adjusted by adjusting how far out of the outer member the funnel is deployed. For example, the outer diameter of the distal-facing outer region may be configured to be adjusted by extending or withdrawing the braided or woven body relative to a distal end opening of the outer member.

For example, an electrical applicator may include: an elongate outer member; a braided or woven body extending or extendable from the elongate outer member; an inner deployment member coupled to a distal attachment site of the braided or woven body, wherein the inner deployment member is configured to be withdrawn proximally to invert the braided or woven body to form a funnel shape having a distal-facing outer region; a first electrically conductive region formed at least partially around the distal-facing outer region, wherein the region of the braided or woven body proximal to the distal-facing outer region are electrically insulated; a second electrically conductive region, wherein the second electrically conductive region is configured to be surrounded by the distal-facing outer region and within the funnel shape when the inner deployment member is withdrawn proximally; and one or more electrical connectors at a proximal end of the device in electrical communication with the first and second conduction regions.

Any of the devices described herein may be part of a system. For example, a system may include any of the electrical applicators described herein; and a pulse generator configured to generate a plurality of electrical pulses having amplitude of at least 0.1 kV and a duration of less than 1000 nanoseconds, the electrical applicator configured to electrically couple to the pulse generator.

Also described herein are devices (e.g., electrical applicators) having more than one funnel. For example, an electrical applicator may include: a braided or woven body; a first funnel shape formed of a first region of the braided or woven body, the first funnel shape having a first distal-facing outer region, a second funnel shape formed of a second region of the braided or woven body, the second funnel shape concentrically surrounding the first funnel shape and including a second distal-facing outer region; a first electrically conductive region formed at least partially around the first distal-facing outer region; and a second electrically conductive region formed at least partially around the second distal-facing outer region.

The first and second funnel shapes may be formed of the same woven or braided material, or they may be formed of separate woven or braided materials. If separate braided or woven materials are used, these separate materials may be coupled together (e.g., by a connector, such as a neck region). In some examples the same region braided or woven material may be used, but different strands (e.g., wires, threads, etc.) forming the braided or woven material may be electrically coupled to the electrical contact and to the electrically conductive region (e.g., electrode) at the distal end.

Any of these devices may include an elongate proximal body extending proximally from the second funnel shape, as mentioned above. For example, the elongate proximal body portion may be a handle and/or connector (e.g., high-voltage connector) as described herein. The devices described herein may include one or more (e.g., two or more, three or more, etc.) electrical connectors at the proximal end region of the elongate proximal body that are in electrical communication with the first and second electrically conductive regions.

In some examples, the device may include an inner deployment member extending within the braided or woven body and coupled to a distal end of the braided or woven body, wherein the inner deployment member is configured to be withdrawn proximally to invert the braided or woven body into the lumen of the braided or woven body to form the first funnel shape or the first and second funnel shapes. The same or a different inner deployment member may be configured to deploy both the first and second funnel shapes. For example, the inner deployment member may be coupled to an attachment region at a distal end of the braided or woven body, and pulling the inner deployment member proximally relative to the proximal end of the braided or woven body (e.g., tube) may form both the first and second funnel shapes. In some examples the device includes (or is configured to operate with) an outer member, wherein the braided or woven body may be slidably positioned within the outer member and configured to extend distally out of the outer member to deploy the first funnel shape and the second funnel shape.

Any of these examples may include a neck member on the braided or woven body that is configured to form the neck of the first funnel shape. In some examples, a second inner deployment member may extend within the braided or woven body and may be coupled to the neck member, wherein the second deployment member is configured to be withdrawn proximally to invert the braided or woven body to form the second funnel shape.

The first region of the braided or woven body may be continuous with the second region of the braided or woven body. The first electrically conductive region and the second electrically conductive region may be coplanar, or not coplanar.

As mentioned, the first conducive region and the second electrically conductive region may be electrically coupled or may be electrically separate.

Thus, these electrical applicators may include two concentrically arranged funnels having electrically conductive regions (electrodes) on their outer surfaces. For example, an electrical applicator may include: a braided or woven body having a lumen extending distally; an inner deployment member extending within the braided or woven body and coupled to a distal end of the braided or woven body, wherein the inner deployment member is configured to be withdrawn proximally to invert a distal end region of the braided or woven body into the lumen of the braided or woven body to form a first funnel shape having a first distal-facing outer region, wherein a second region of the braided or woven body is configured to invert into the lumen of the braided or woven body to form a second funnel shape that concentrically surrounds the first funnel shape and includes a second distal-facing outer region; a first electrically conductive region formed at least partially around the first distal-facing outer region; a second electrically conductive region formed at least partially around the second distal-facing outer region; and a third conductive region distal to the distal end of the braided or woven body, wherein the third conductive region is configured to be surrounded by the second distal-facing outer region and within the second funnel shape.

Any of the devices (and/or systems) described herein may be configured to include pre-formed (rather than deployable) funnels. Thus, these devices may not necessarily include an inner deployment member, as described above. In some examples these devices may instead include a funnel that is either pre-expanded, or that is collapsed and held within a catheter from which it may be extended distally to expand. These devices may optionally include a second electrically conductive region within the funnel shape of the device.

For example, an electrical applicator may include: an elongate outer member; a braided or woven body within the elongate outer member and configured to be extendable distally out of the elongate outer member to expand into a funnel shape having a distal-facing outer region; a first electrically conductive region formed at least partially around the distal-facing outer region of the funnel shape; and a second electrically conductive region within the funnel shape and surrounded by the distal-facing outer region.

As described above, any of these devices may include an elongate proximal body extending proximally from the funnel shape. In some examples, the device includes a pair of electrical connectors at a proximal end region of the elongate proximal body in electrical communication with the first and second electrically conductive regions.

The braided or woven body may comprise a conductive wire. The first electrically conductive region may be formed of an uninsulated portion of the conductive wire forming the braided or woven body. The portion of the braided or woven body proximal to the distal-facing outer region may be electrically insulated.

In any of these examples, the second electrically conductive region may be configured to radially expand when the braided or woven body expands into the funnel shape. The second electrically conductive region (e.g., electrode) may be coplanar with the first electrically conductive region (e.g., on the distal-facing end region of the funnel). Any of these devices may include an elongate positioning member extending proximally from the second electrically conductive region within the braided or woven body that is configured to allow adjustment of the longitudinal position of the second electrically conductive region relative to the distal-facing outer region of the funnel shape.

In general, the outer diameter of the distal-facing outer region may be configured to be adjusted by extending or withdrawing the braided or woven body relative to a distal end opening of the outer member. Further, in any of the examples, the apparatuses/devices of the present disclosure may comprise or be used with a centering guide/centering feature configured to improved positioning and centering of the device relative to the target tissue.

For example, described herein are electrical applicator devices comprising: a braided or woven body; a first region of the braided or woven body forming or configured to form a first funnel shape, the first funnel shape having a first distal-facing outer region, a second region of the braided or woven body forming or configured to form a second funnel shape, the second funnel shape having a second distal-facing outer region; a first electrically conductive region at least partially around the first distal-facing outer region; and a second electrically conductive region at least partially around the second distal-facing outer region. The first funnel shape may concentrically surround the second funnel shape, or the second funnel shape may concentrically surround the first funnel shape. Any of these devices may include an elongate proximal body extending proximally from the braided or woven body. Any of these devices may include a pair of electrical connectors at a proximal end region of the elongate proximal body in electrical communication with the first and second electrically conductive regions.

In some examples, the device may include an inner deployment member extending within the braided or woven body and coupled to a distal end of the braided or woven body, wherein the inner deployment member is configured to be withdrawn proximally to invert the braided or woven body to form at least one of the first funnel shape and the second funnel shape, or both the first funnel shape and the second funnel shape.

Any of these devices may include a third conductive region surrounded by the first distal-facing outer region and within the first funnel shape. The apparatus may apply energy between electrodes on the distal-facing ends of the first, second or third funnel shapes (e.g., between first and second, between first and third, or between second and third). The first electrically conducive region and the second electrically conductive region may be electrically coupled.

Any of these devices may include an outer member, wherein the braided or woven body is slidably positioned within the outer member and configured to extend distally out of the outer member to deploy the first funnel shape and the second funnel shape. The device may include a neck member on the braided or woven body between the first region of the braided or woven body and the second region of the braided or woven body.

A second inner deployment member may be included and configured to be withdrawn proximally to invert the braided or woven body to form the second funnel shape.

The first region of the braided or woven body may be continuous with the second region of the braided or woven body. In some examples, the first electrically conductive region and the second electrically conductive region are not coplanar.

Also described herein are methods of using any of these devices and systems described herein. For example, a method of treating tissue using any of these devices may include: positioning an electrical applicator against a target tissue so that a first electrically conductive region on a distal-facing outer region of a braided or woven funnel shape contacts the target tissue and a second electrically conductive region that is surrounded by the distal-facing outer region of the braided or woven funnel shape contacts the target tissue; and applying a plurality of electrical pulses having an amplitude of at least 0.1 kV and a duration of less than 1000 nanoseconds to the target tissue from the first and second electrically conductive regions

The method may also include deploying the electrical applicator prior to positioning the electrical applicator, for example, by withdrawing an inner deployment member of the electrical applicator proximally to invert a distal end region of a braided or woven body of the electrical applicator to form the funnel shape having the distal-facing outer region.

Any of these methods may also include adjusting the longitudinal position of the second electrically conductive region using an elongate positioning member extending proximally from the second conductive member through the braided or woven body. For example, a method as described herein may include deploying the braided or woven funnel shape out of an outer member, such as outer catheter.

The methods described herein may also or alternatively include adjusting the outer diameter of the distal-facing outer region by extending or retracting the braided or woven funnel shape out of the outer member.

The plurality of electrical pulses may be applied from the electrical applicator in a monopolar configuration. Alternatively, the plurality of electrical pulses may be applied from the electrical applicator in a bipolar configuration.

In any of the methods described herein, applying the plurality of electrical pulses may comprise applying the plurality of electrical pulses as part of a single shot. For example, these methods may include applying a single shot of treatment to the target tissue, wherein the plurality of electrical pulses are applied circumferentially to the target tissue (e.g., in a ring around or against the target tissue), and removing the electrical applicator from the target tissue after applying the plurality of electrical pulses. In some cases the electrical applicator may be completely removed from the body or repositioned against another target tissue, e.g., in another portion of the body. Thus, the apparatuses described herein may advantageously apply treatment to a large region of target tissue in a single shot, without requiring multiple steps of repositioning and applying treatment to a target tissue.

These methods may be used to treat a target including one or more of: vein, artery, vessel, heart, trachea, pharynx, larynx, bronchi, ureter, urethra, fallopian tubes, cervix, uterus, intestine, pancreas, pancreatic duct, liver and hepatic ducts, rectum, esophagus, stomach, nasal cavity, seminal vesicles, bronchi, and vocal cords/vocal folds. In one example the methods may be methods of treating endometriosis, and the tissue may be female reproductive system tissue (e.g., the ovaries, fallopian tubes, ligaments that support the uterus (uterosacral ligaments), posterior cul-de-sac, i.e., the space between the uterus and rectum, anterior cul-de-sac, i.e., the space between the uterus and bladder, outer surface of the uterus, lining of the pelvic cavity, and in some cases the intestines, rectum, bladder, vagina, cervix, and/or vulva.

For example, a method of treating tissue may include: withdrawing an inner deployment member of an electrical applicator proximally to invert a distal end region of a braided or woven body of the electrical applicator to form a funnel shape having a distal-facing outer region; positioning a first electrically conductive region on the distal-facing outer region against a target tissue so that a second electrically conductive region that is surrounded by the distal-facing outer region within the funnel shape also contacts the target tissue; and applying a plurality of electrical pulses having an amplitude of at least 0.1 kV and a duration of less than 1000 nanoseconds to the target tissue from the first and second electrically conductive regions.

The apparatuses described herein may be configured for manual or automated (e.g., robotic assisted) control. In some variations these apparatuses may be integrated into systems that are configured to be mounted onto or coupled to a movable (e.g., robotic) arm of a robotic system, such as robotic medical treatment system or robotic surgical system. For convenience of description the present disclosure may refer to these as robotic systems, however, it should be understood that such robotic systems are intended to cover any robotic medical treatment system (including for cosmetic applications) and may include robotic systems having guidance. In some variations instruments can be guided and controlled by the robotic system during a surgical or other medical procedure. For example, the devices described herein may be used through one or more operating channels of a robotic system.

As mentioned, any of these apparatuses may be configured so that the proximal end of the applicator device is adapted to be coupled to a robotic arm, for example, for computer-controlled activation of the first and second electrodes. Alternatively or additionally the proximal end of the applicator device may be adapted to couple to a handle of the pulse generator which may in turn by adapted for connection to a robotic arm.

The apparatuses described herein may generally be configured safely and reliably to deliver microsecond, nanosecond, picosecond, etc. pulses, and may include an electric field with a pulse width of between 0.1 nanoseconds (ns) and less than 1000 nanoseconds, or shorter, such as 1 picosecond, which may be referred to as sub-microsecond pulsed electric field. This pulsed energy may have high peak voltages, such as 1 to 5 kilovolts per centimeter (kV/cm), 10 kV/cm, 20 kV/cm, 100 kV/cm or higher. Treatment of biological cells may use a multitude of periodic pulses at a frequency ranging from 0.1 per second (Hz) to 10,000 Hz, and may trigger regulated cell death (e.g., apoptosis), for example, in the diseased tissue, unwanted tissue or abnormal growth, such as cancerous, precancerous or benign tumors and other target tissue. Selective treatment of such tumors, lesions or target tissue with high voltage, sub-microsecond pulsed energy can induce regulated cell death within the tumor or target tissue cells without substantially affecting normal cells in the surrounding tissue due to its non-thermal nature. A subject may be a patient (human or non-human, including animals). A user may operate the apparatuses described herein on a subject. The user may be a physician (doctor, surgeon, etc.), medical technician, nurse, or other care provider.

Thus, the application of high voltage, fast (e.g., microsecond or submicrosecond) electrical pulses may include applying a train of electrical pulses having a pulse width, for example, of between 0.1 nanoseconds (ns) and 1000 nanoseconds. Applying high voltage, fast electrical pulses may include applying a train of sub-microsecond electrical pulses having peak voltages of between, for example, 1 kilovolt per centimeter (kV/cm) and 100 kV/cm. Applying high voltage, fast electrical pulses may include applying a train of sub-microsecond electrical pulses at a frequency, for example, of between 0.1 per second (Hz) to 10,000 Hz.

Any of these apparatuses may be used with a pulse generator. For example, described herein are systems for treating tissue that may include: an elongate applicator tool as described herein (e.g., an elongate body having a distal end region that articulates, from which one or more electrodes are configured to extend), a connector, e.g., a high voltage connector adapted to couple the elongate applicator tool to a pulse generator; and a pulse generator configured to generate a plurality of electrical pulses having amplitude of at least 0.1 kV and a duration of less than 1000 nanoseconds, the pulse generator comprising a port configured to connect to the high voltage connector.

In general, any of these apparatuses may be used in conjunction with or be a part of the system that includes a pulse generator configured to generate a plurality of electrical pulses having amplitude of at least 0.1 kV and a duration of less than 1000 nanoseconds, the pulse generator may be configured to connect to the apparatus with a high voltage connector.

The apparatuses described herein may additionally or alternatively be configured so that they may operate with the braided or woven body extending expanded radially outward but without inverting to form a funnel shape. The inner deployment region may extend within the braided or woven body and be coupled to a distal attachment site of the braided or woven body, so that proximally retracting the inner deployment member may radially expand the braided or woven body. The inner deployment member may be extended distally to collapse (e.g., reduce the radial expansion of) the braided or woven body. One or more conductive regions on the outer surface of the braided or woven body (including a central region, or one or more peripheral regions) may be included and may be used to apply energy, including nanosecond pulsed electrical fields, as described herein. Any of these devices may still include one or more central electrically conductive regions (electrodes) that may be on the inner deployment region and may be positioned distally of the electrically conductive region(s) on the braided or woven body, and/or they may be positioned within the braided or woven body. The central electrode(s) may be used for the application of bipolar energy from the outer surface of the braided or woven body.

Devices in which the braided or woven body is configured to radially expand without inverting may be used to apply energy, for example, against the sides of a body lumen in which the distal end of the device is navigated. The expanded braided or woven body may be driven against the wall(s) of the lumen and may conform to the wall(s) and make good electrical contact. In some examples, nanosecond pulsed electrical field may be applied, as described herein.

In these examples, the inner deployment member may be limited (e.g., by a stop) to prevent the braided or woven body from inverting. In some variations the apparatus may be configured to operate in both configurations, with the braided or woven body radially expanded but not inverted (e.g. to apply energy to the sides of a body lumen), and with the braided or woven body inverted at the distal end to form a funnel-shape as described above. Thus, in some examples, the inner deployment member may engage a releasable stop to prevent the braided or woven body from inverting into the funnel and this stop may be released to then allow it to be inverted.

### BRIEF DESCRIPTION OF THE DRAWINGS

The novel features of the present disclosure are set forth with particularity in the claims that follow. A better understanding of the features and advantages of the present disclosure will be obtained by reference to the following detailed description that sets forth illustrative embodiments, in which the principles of the disclosure are utilized, and the accompanying drawings of which:

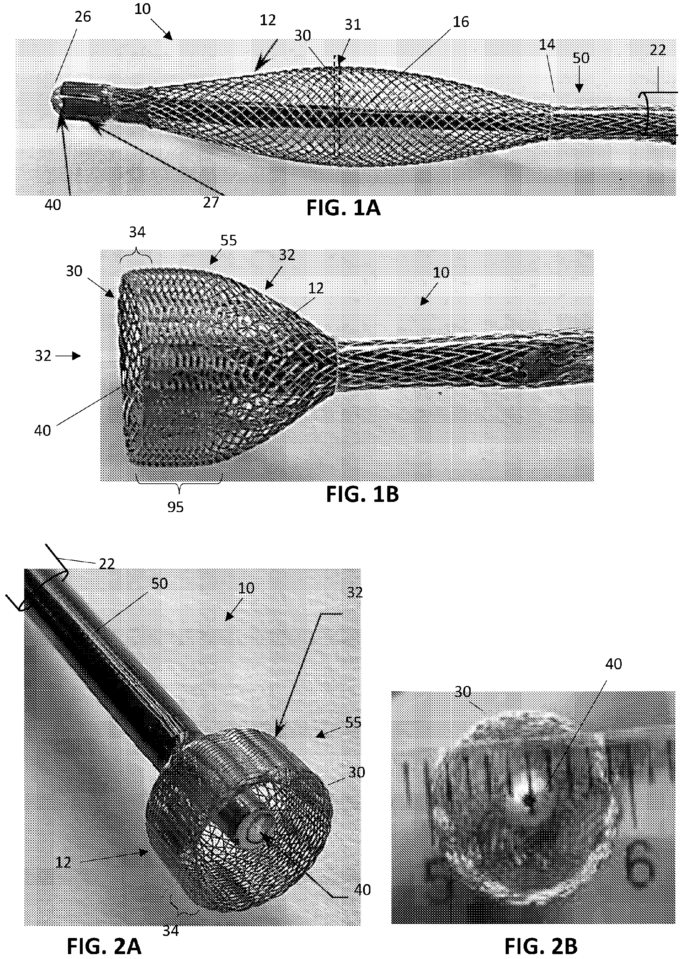

FIGS. 1A and 1B illustrate one example of an electrical applicator (e.g., applicator device) having a funnel-shaped electrode as described herein. FIG. 1A shows the woven body of the electrical applicator in an un-deployed configuration, extending distally from out of an outer member, with the braided or woven mesh (shown here as a woven mesh) extending in a tube that self-expands slightly extending distally-to-proximally. FIG. 1B shows a side view of the electrical applicator of FIG. 1A in a deployed configuration, forming a funnel shape having a distally facing outer region.
FIGS. 2A and 2B show another example of an electrical applicator having a funnel-shaped electrode. In FIG. 2A, the electrode is shown as a deployed electrical applicator, such as that shown in FIG. 1B, from a perspective view. FIG. 2B shows the applicator device of FIG. 2A from an end view.

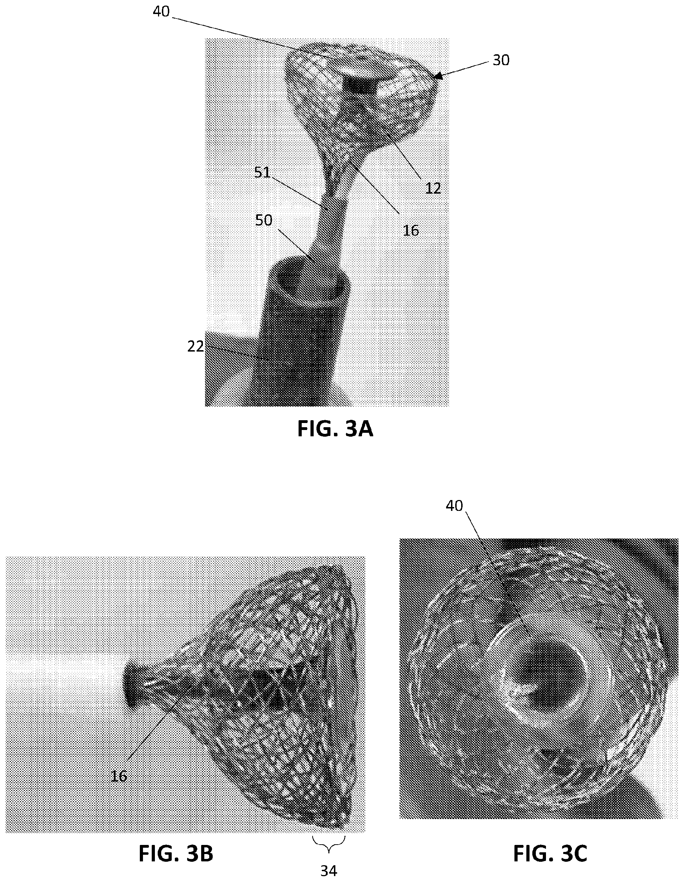

FIGS. 3A-3C illustrate another example of an electrical applicator having a funnel-shaped electrode. FIG. 3A shows the electrical applicator in a deployed configuration, extending from an elongate outer catheter. FIG. 3B is a side view of the electrical applicator, and FIG. 3C is an end view, showing the distal-facing end of the deployed electrical applicator.

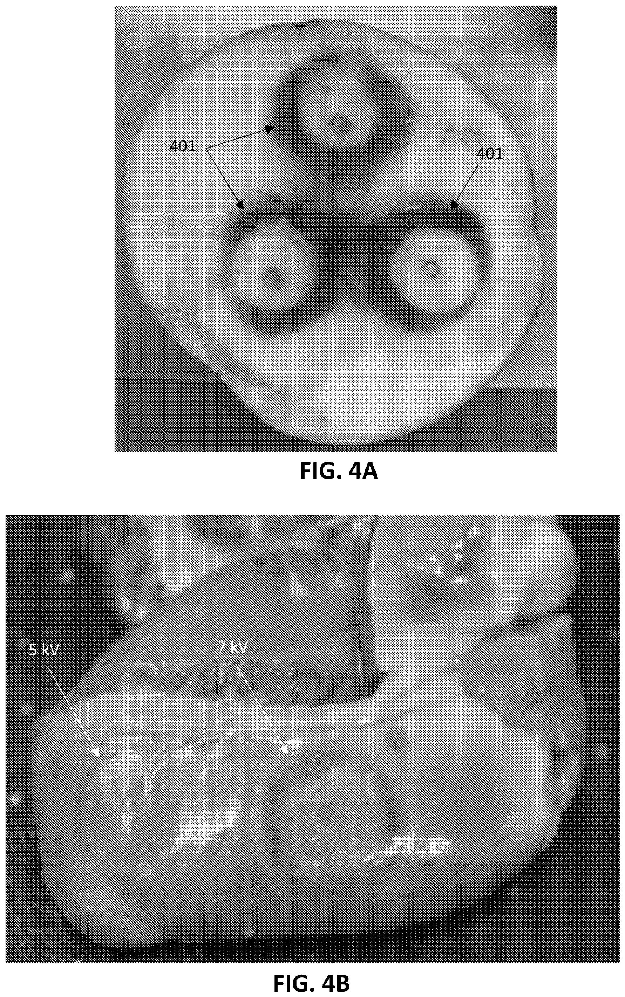

FIG. 4A is an image showing experimental results of an electrical applicator similar to that shown in FIGS. 1A-1B and 2A-2B. In this example the electrical applicator was operated as a monopolar electrode on a tissue model, e.g., sliced potato (energy was applied to multiple sites). In FIG. 4A the tissue model is shown 23 hours after applying 100 pulses of 200 ns (8 Hz) at 5 kV.
FIG. 4B is an image showing experimental results of an electrical applicator similar to that shown in FIGS. 1A-1B and 2A-2B. In this example the electrical applicator was operated as a monopolar electrode on animal tissue (energy was applied to multiple sites with different parameters). In FIG. 4B, the tissue is shown 4-4.5 hours after applying 100 pulses of 200 ns (6 Hz) at either 5 kV or 7 kV.

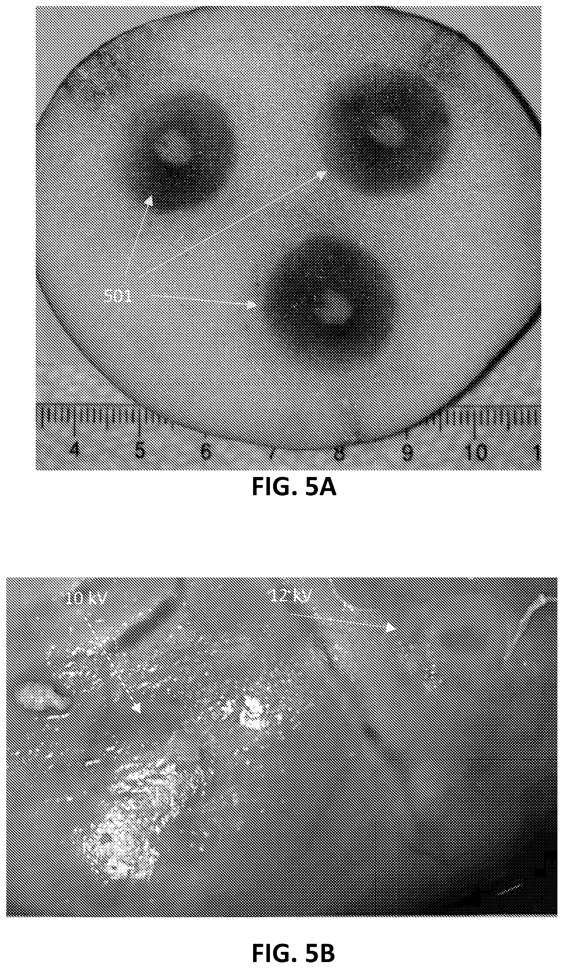

FIG. 5A is an image showing experimental results of an electrical applicator similar to that shown in FIGS. 1A-1B and 2A-2B operated in this example as a bipolar electrode. In FIG. 5A, the tissue model is shown 23 hours after applying 100 pulses of 200 ns (8 Hz) at 12 kV.
FIG. 5B is an image showing experimental results of an electrical applicator similar to that shown in FIGS. 1A-1B and 2A-2B operated as a bipolar electrode on animal tissue (energy was applied to multiple sites with different parameters). In FIG. 4B, the tissue is shown 5-4.5 hours after applying 100 pulses of 200 ns (6 Hz) at either 10 kV or 12 kV.

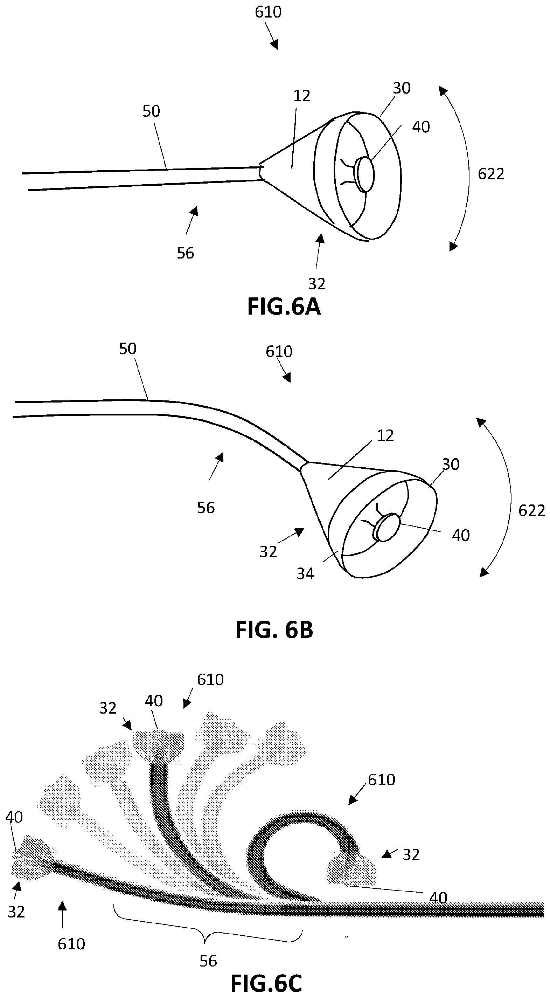

FIGS. 6A-6C illustrate another variation of an electrical applicator as described herein, in which the distal-facing funnel shaped electrode region is configured to be steered, e.g., using one or more tendons. FIG. 6A shows the electrical applicator in a neutral position, deployed with the funnel shaped electrode region facing distally in the long axis of the device. FIG. 6B shows the electrical applicator steered with the funnel shape pointing down. FIG. 6C shows an example of an electrical applicator bending at the distal end region to steer or orient the distal-facing funnel shaped electrode region.

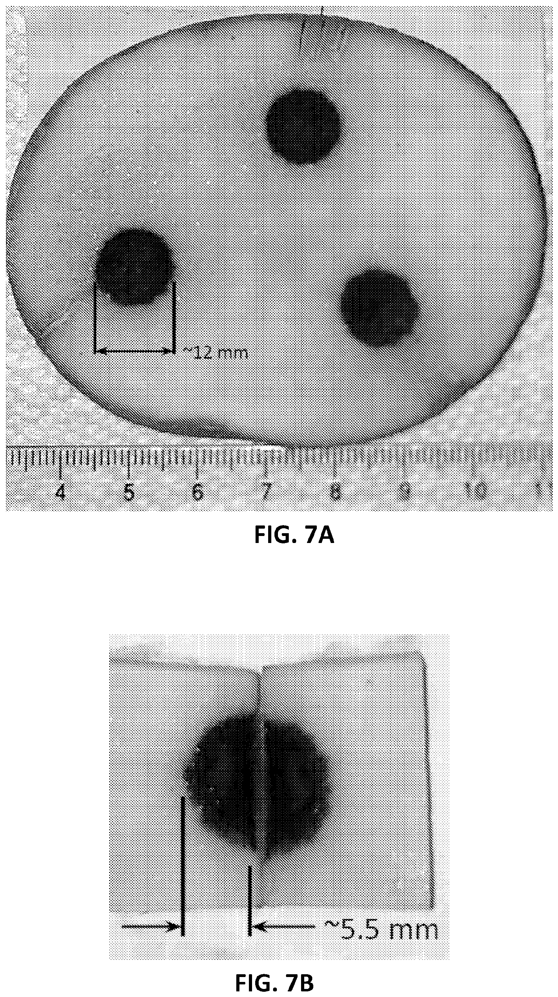

FIGS. 7A-7B illustrate the effects of an electrical applicator of the present disclosure used to apply nanosecond pulsing to a test tissue (e.g., potato). In FIG. 7A, the surface of the test tissue is shown 24 hours after applying nanosecond pulsing. FIG. 7B shows both side of a section through the test tissue, showing the penetration into the tissue, of the same sample tissue of FIG. 7A.

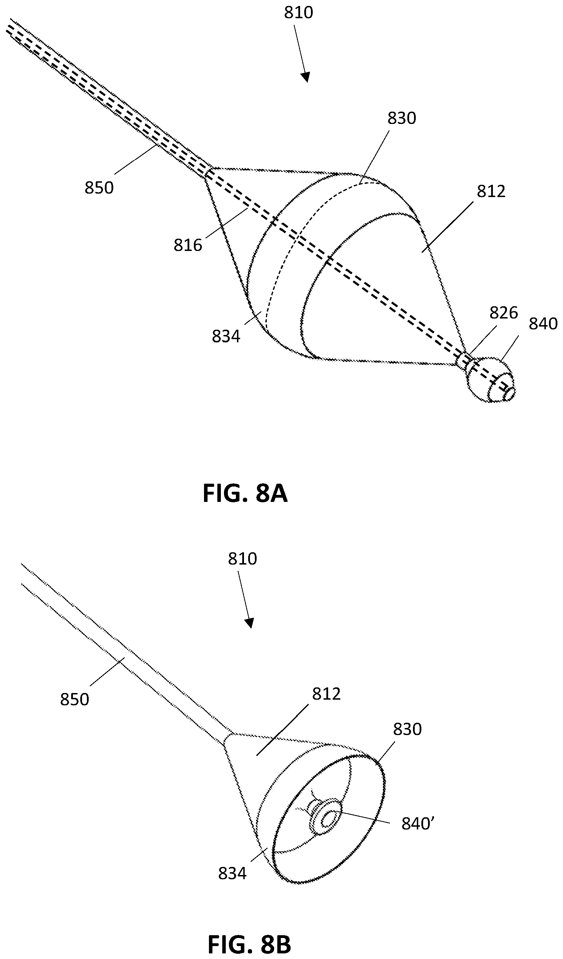

FIGS. 8A-8B illustrate another example of an electrical applicator having a funnel-shaped electrode, in which the second electrically conductive region (e.g., second electrode) within the funnel shape is configured to collapse when the device is deployed. FIG. 8A shows the device in an un-deployed configuration. FIG. 8B shows the device in a deployed configuration.

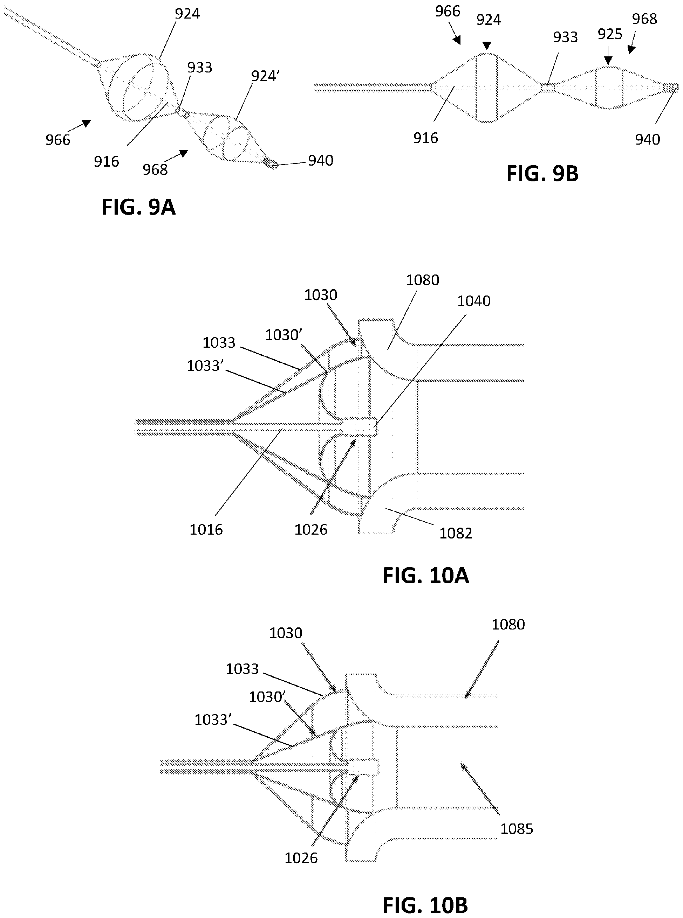

FIGS. 9A-9B illustrate another example of an electrical applicator having a pair of funnel-shaped electrodes, shown in an un-deployed configuration. In FIG. 9A the un-deployed elongate braided or woven material is shown in a perspective view expanded (e.g., outside of an outer member, e.g. catheter) but prior to withdrawing the inner deployment member proximally to form a pair of concentric funnels, as shown below in FIGS. 10A-10B. FIG. 9B shows a side view of the un-deployed device of FIG. 9A.
FIGS. 10A-10B show examples of sections through electrical applicators each having a pair of funnel-shaped electrodes being used to treat tissue. FIG. 10A shows an electrical applicator including a pair of funnels, each with electrodes on them, used to treat tissue (e.g., for antrum ablation). FIG. 10B shows another example of an electrical applicator having a pair of funnels with electrodes on the distal-facing end regions, similar to that shown in FIG. 10A, being used, e.g., for ablating antrum (including, but not limited to ablating antrum-ostium).

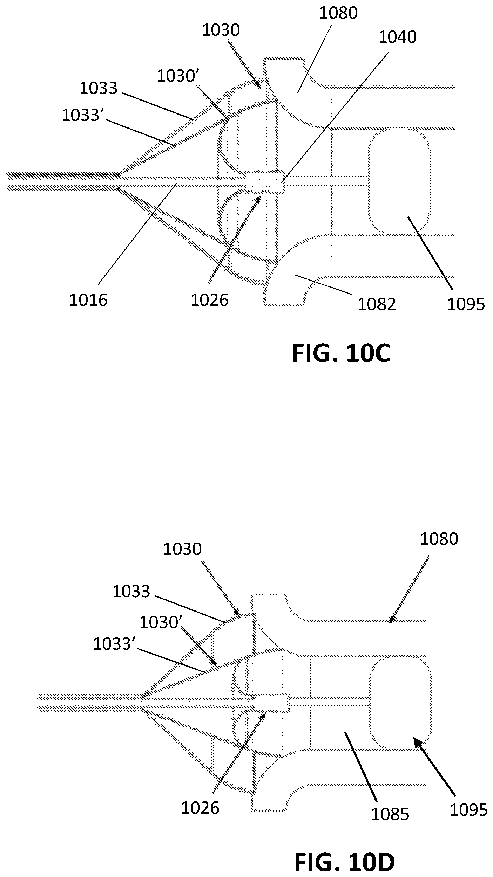

FIGS. 10C and 10D illustrate examples of applicators similar to those shown in FIGS. 10A and 10B, but with a centering guide extending distally from the device.

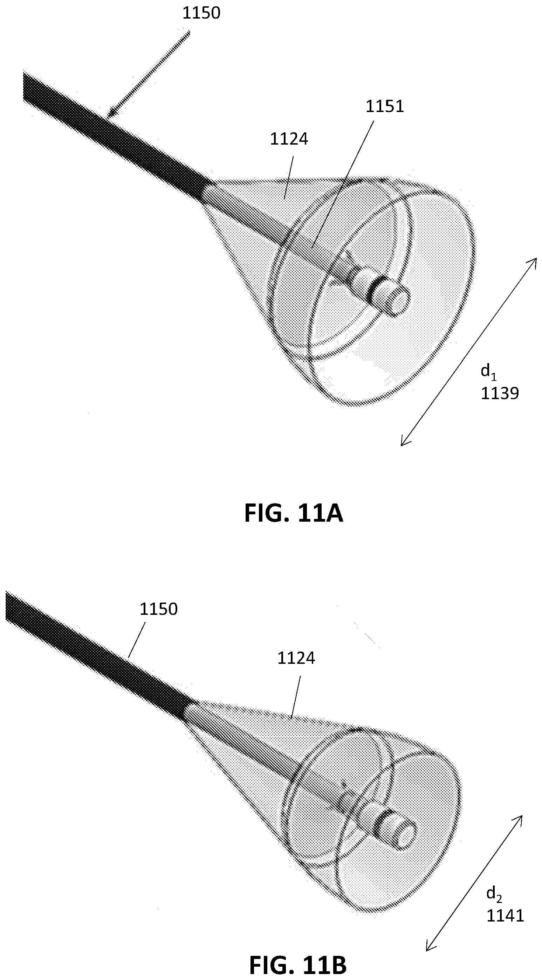

FIG. 11A shows one example of an electrical applicator having a funnel-shaped electrode having first outer diameter.
FIG. 11B shows the device of FIG. 11A partially retracted into an outer catheter so that the outer diameter is constricted to a smaller outer diameter, as shown.

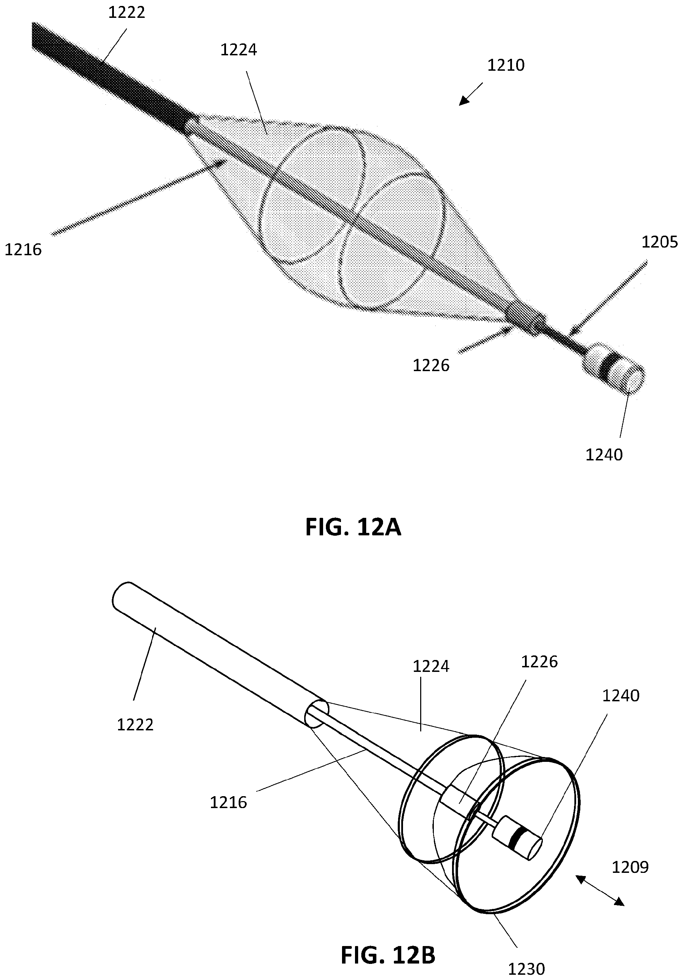

FIGS. 12A-12B show an example of an electrical applicator having a funnel-shaped electrode with a second electrically conductive region (electrode) that may be adjustably positioned relative to the longitudinal position. FIG. 12A shows the electrical applicator in the undeployed configuration; FIGS. 12B shows the device in the deployed configuration, with the braided or woven body inverted back over itself, to form the funnel shape, with one or more electrodes on the distal-facing end region.

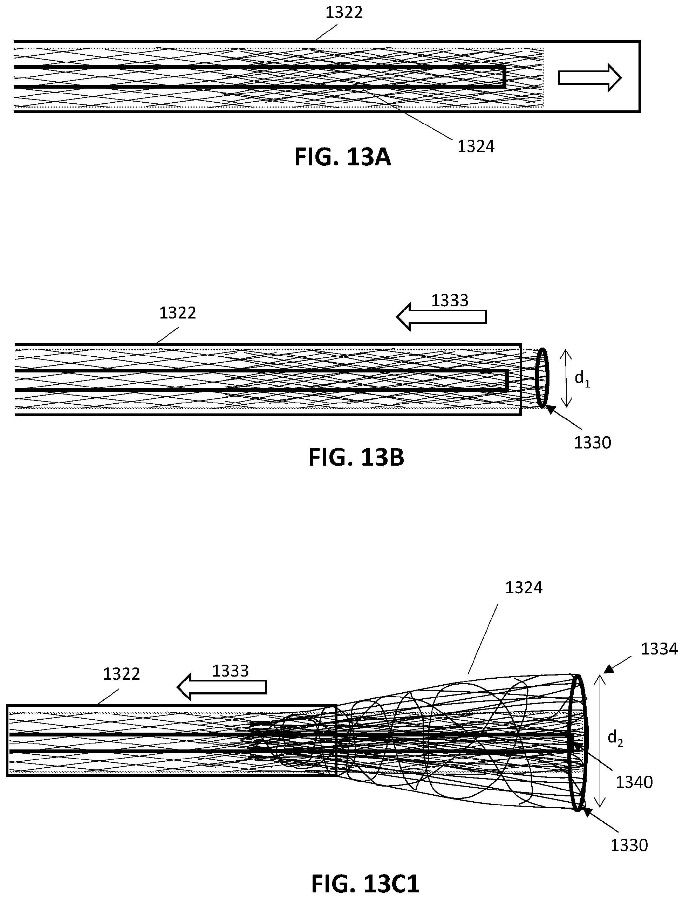

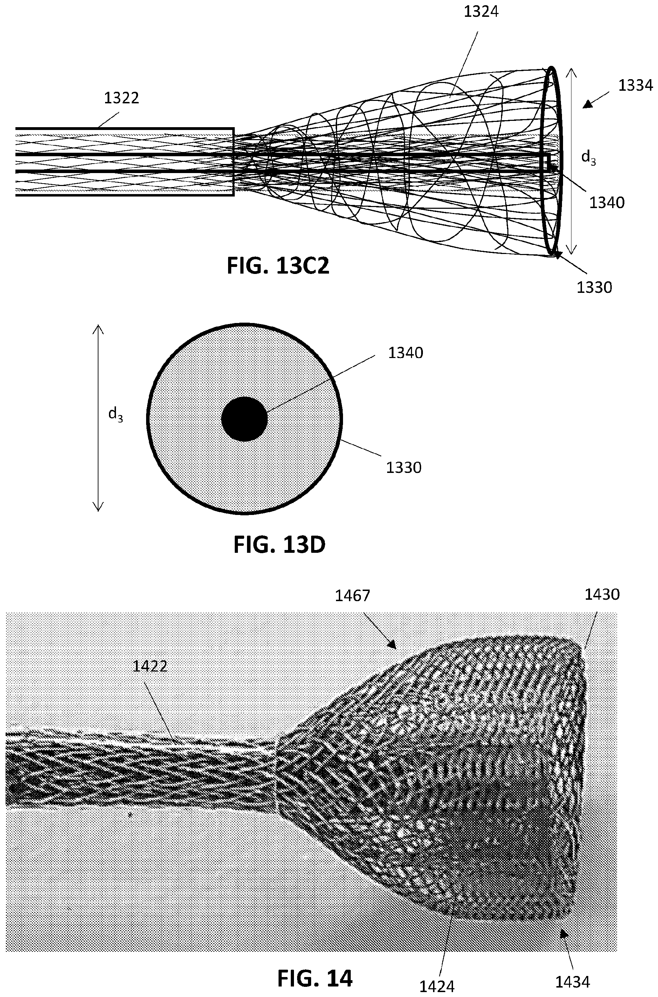

FIGS. 13A-13D illustrate another example of a funnel-shaped electrical applicator in which the braided or woven material forming the funnel is preformed into the funnel shape but is held in an undeployed collapsed configuration (as shown in FIGS. 13A-13B) and is extended distally out of a catheter and allowed to expand, as shown in FIGS. 13C1 and 13C2. Fig. 13D shows a distal end view of the device of FIGS. 13A-13C.
FIG. 14 is an example of a funnel-shaped electrical applicator similar to that shown in FIGS. 13A-13D.

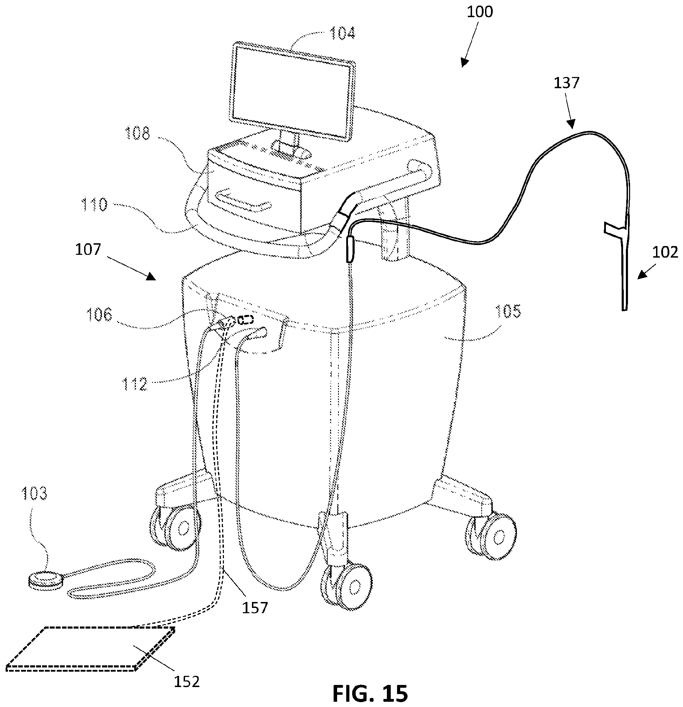

FIG. 15 illustrates one example of a system, including an electrical applicator of the present disclosure and a pulse generator, for delivery of high voltage, fast pulsed electrical energy.

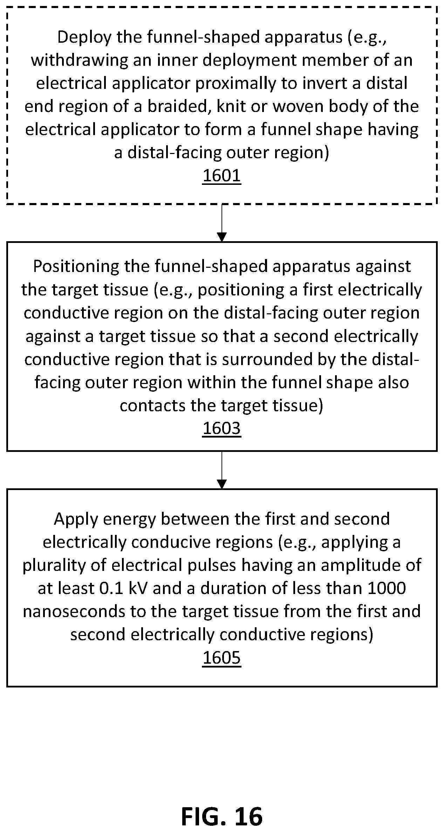

FIG. 16 schematically illustrates one example of a method of treating a patient by applying energy using any of the apparatuses described herein.

### DETAILED DESCRIPTION

Described herein are applicators for delivering electrical energy to a tissue having a funnel shape that may include one or more electrodes on a distal-facing end region of the funnel shape. An additional electrode may be positioned within the opening of the distal-facing funnel shape. These applicators may be configured for monopolar or bipolar operation and may be used to treat tissue, including in particular for the delivery of nanosecond pulsed energy, such as high voltage sub-microsecond (e.g., nanosecond, picosecond, etc.) electrical energy to target tissue.

The electrical applicators described herein may generally include a body that forms or is configured to be deployed to form, a distal facing funnel shape. This body may be formed of a braided and/or woven material. The braided or woven material may be an electrically conductive material that may be insulated over most of the body but may be exposed to form one or more electrically conductive regions (e.g., electrodes), such as on the distal facing end region of the funnel shape. The material may be, for example, a conductive polymer, a shape memory alloy (e.g., a nickel titanium material such as Nitinol), stainless steel, etc. The material may be insulated with a polymeric material. The material may be formed of one or more stands or filaments, or bundles of strands or filaments (including wires). In some variations the braided or woven material is formed of a non-conductive material (e.g. a polymeric material) into which one or more strands of conductive material have been included for forming the distal electrically conductive regions. In some variations most or all of the braided or woven material is formed of a conductive material. Although the majority of the examples provided herein include a braided or woven material forming the body (e.g., forming the funnel shape), any of the examples provided herein may instead use a sheet of material, such as a laser-cut sheet of material. The braided and/or woven materials may be fibrous materials (including natural fibers, synthetics fibers, etc.), polymeric materials, or the like. For example, the material (e.g., strands) forming the woven, braided or knitted material may be one or more of: monofilament polymer, multifilament polymer, NiTi filament, NiTi tube with radiopaque metallic center, Cobalt chromium alloy filament, Cobalt chromium alloy tube with radiopaque metallic center, Nylon, Polyester, Polyethylene terephthalate, and Polypropylene. A sheet of material (e.g. a solid sheet of material) may be one or more of: polymeric material (e.g., PTFE), silicone materials, polyurethanes, shape memory alloys, stainless steels, etc. The sheets may be extruded, glued, or the like. The sheets may be cut to form pores and/or projections. For example, the sheets may include one or more laser-cut projections. Any of these apparatuses may be coated with a hydrophilic and/or hydrophobic coating, and/or may include pores.

In some variations the body that forms the funnel shape may be woven. A woven body may include a plurality of strands (e.g., between 8-100, between 10-70, between 20-60, etc.). As mentioned, some or all of the strands may be formed of an electrically conductive material, which may be insulated. Insulation may be removed over the region forming the electrically conductive region, or electrode, such as at the distal-facing end region, and in some examples an outer or end portion of the distal-facing end region. Similarly, the body may be formed of a braided material, e.g., a plurality of strands that are braided to form the material of the body. The woven body regions may be equivalently formed by a braid (and be referred to as "braided" body regions). In some variations the body may be formed of a knitted material, such as a knitted filament. Thus, in general all of these braided, knitted, woven body regions (including those from the laser-cut sheets) may be referred to as "braided or woven" body or "braided or woven" material.

The electrical applicators described herein may include an elongate proximal region that may be hand-held (e.g., by a user) or it can be coupled to a movable arm of a robotic system, and its operation may be at least partially automated or fully automated, including computer controlled.

Any of the electrical applicators described herein may be configured to deploy from an undeployed state in which electrical applicator has a narrower outer diameter and/or may be held within an outer member, such as outer catheter or cannula, to a deployed state in which the electrical applicator is expanded to form a funnel shape. In some examples the electrical applicator includes a braided or woven body that is shaped like a tube in which the proximal end is gathered, and the distal end is gathered. The proximal end may be continuous with or coupled to an elongate member, such as a cannula or catheter. The distal end may be coupled to an elongate puller or pusher (e.g., a microcatheter, guidewire, etc.) that passes within the elongate body to which the braided or woven body is attached; this puller may be referred to as an inner deployment member.

For example, FIG. 1A illustrates an example of an electrical applicator 10 that is configured to form a funnel body, shown in an un-deployed (or, as described below, partially deployed) state. In this example, the braided or woven body 12 is formed of a woven shape memory alloy material (e.g. NITINOL) that may shape-set to have an expanded configuration, e.g., with the middle region of the un-inverted braided or woven body flared out. The middle region 31 of the braided or woven body may be configured as an electrically conductive region (e.g., an electrode), by including one or more conductive materials in this region. The first electrically conductive region may be referred to as an electrode or as a braided electrode. In this example the first electrically conductive region may be formed from some or all of the wires of the braided or woven body, which may be insulated in the proximal and distal regions but may be electrically exposed over this middle region 31 of the braided or woven body. The electrically exposed regions may form one or more electrically conductive regions. The electrically exposed regions may contact each other to form a single contiguous electrode, or the electrically exposed regions may be separated from each other by insulated regions to form multiple electrodes on the braided or woven body. For example, the first electrically conductive region may be formed of a plurality of exposed (un-insulated) regions of electrically conductive wire, which may be the wire forming the braided or woven body or may be added to the wire(s) forming the braided or woven body. As shown in FIGS. 1A-1B, when the wires at the mid-region 31 of the braided or woven body are exposed around all or part of the periphery of this region, when the funnel-shape is formed (as described below and illustrated in FIG. 1B) the distal-facing end of the funnel shape may form a conductive region. The rest of the braided or woven body can be electrically insulated, e.g., by insulating each individual wire with an electrically insulating coating, or by collectively insulating them. This may prevent shorting of the applied electrical energy, which may contact other (non-target tissue) and/or blood or bodily fluid, e.g., as may be the case when used, e.g., for endocardial use or within a body lumen.

In any of the device described herein one or more (e.g., two, three, four, five, six, etc.) electrically conductive regions may be formed on or around the braided or woven body when it is inverted as shown in FIG. 1B and 2A. Each region may be formed from the same wires forming the braided or woven body or they may be formed of an additional one or more wires that are coupled to the braided or woven body. Each electrically conductive region (electrode) may be electrically coupled with an electrical contact at a proximal end region of the device. For example, in variations in which the electrically conductive region(s) on the braided or woven body are formed form the wires forming the braided or woven body, these wires may be electrically coupled at the proximal end of the braided or woven body to an electrical connector at or near the proximal end of the device. In some examples the wires forming the braided or woven body may extend down the length of the elongate proximal body 50 extending proximally from the funnel shape.

In FIG. 1A the distal end of the braided or woven body is gathered and coupled to a collet that is coupled to the inner deployment member 16. The proximal end 14 of the braided or woven body is also gathered and coupled to an elongate proximal body 50 that extends proximally, within the outer catheter 22 in FIG. 1A and 2A. In any of these examples, another electrically conductive region, e.g., an electrode or center electrode, may be included within the opening formed by the funnel shape. In FIG. 1A, the electrode 40 may be coupled (e.g., mechanically coupled) to the gathered distal end (e.g., the distal collet 26) of the braided or woven body. This electrode may be referred to as a second electrically conductive region (or central electrically conductive region, or center electrode). In some examples the central electrically conductive region 40 is configured to be positioned in the same plane as the distal-facing opening of the funnel shape, and therefore the same plane as the one or more electrodes 30 on and around the distal-facing outer region of the funnel shape 55. The central electrically conductive region 40 may be fully electrically insulated but for the distal-facing surface.

The entire electrical applicator device may be configured for monopolar and/or bipolar operation. For example, the electrical applicator device may be configured for monopolar operation by electrically coupling the one or more electrically conductive regions at least partially around the distal-facing outer region with the center electrode 40. For example, the electrically conductive region may be electrically coupled to the wires forming the braided or woven body 12 (monopolar configuration). Alternatively the central electrode may be separately electrically connected to the pulse generate and may be configured to have an opposite polarity (bipolar configuration).

In the example shown in FIGS. 1A-1B the one or more electrically conductive regions on the braided or woven body are formed at the end of the funnel-shape, e.g., on the distal end region 34. In some variations, the electrically conductive region(s) may extend slightly proximally, and/or may be on the distal-facing edge and/or may extend into the funnel shape. In some examples the one or more electrically conductive region(s) are on the distal-facing edge of the distal-facing outer region.

In FIG. 1A the middle region is also configured to invert when the inner deployment member 16, which extends through the braided or woven body, is pulled proximally, as shown in FIG. 1B, forming a funnel shape or funnel 55 having a distal-facing distal end region. The first electrically conductive region may extend around the entire braided or woven body, or it may only extend partially around the distal end region 34 of the funnel shape 55.

The electrical applicator of FIGS. 1A-1B may be configured for bipolar operation. In FIG. 1A, the applicator is shown pre-deployment, with the braided or woven body 12 extending distally and proximally. An inner deployment member 16 is coupled to the distal end of the braided or woven body and may also house the electrical connection to the center electrode 40. The sides 27 of this electrode are insulated. The device may be fully deployed by pulling proximally on the inner deployment member, as shown in FIG. 1B.

FIG. 2A shows a front perspective view of another example of an electrical applicator device, similar to that shown in FIG. 1A-1B, including a braided or woven body 12 that has formed a funnel-shaped body. The distal end 26 may be pulled (e.g., by pulling on an inner deployment member to deploy the funnel shape) to invert this distal end of the braided or woven body into itself. In this example although the shape is funnel-shaped (which may also be referred to as flared outwards, or trumpet-shaped), the inside of the funnel shape 55 is at least partially blocked by the end of the braided or woven body that was inverted into itself. FIG. 2B shows an end view into the distal-facing opening formed by the funnel.

In any of these examples the funnel shape may be compressed by pulling proximally, to form a more cylindrical distal end region 34, at the end of a high column strength region 95 (as shown in FIG. 1B) that may provide a high amount of column strength, improving the ability of the device to push against tissue. Thus, the funnel shape 55 may be held under compression either before and/or after deployment. The wires forming the funnel body (the braided or woven body) may stack up against each other. FIGS. 2A and 2B illustrate top perspective and front (end) views, respectively with the funnel shape shown compressed to form this high column strength region 95.

The distal end region 34 of the funnel shape 55 in FIGS. 2A-2B also includes one or more electrodes 30 arranged at least partially around the distal-facing end. In some variations the central conductive region 40 may be open (e.g., may be ring-shaped, to allow one or more elongate members (e.g., guidewires, scopes, etc.) to pass through the middle of the device.

FIGS. 3A illustrates another example of an electrical applicator shown in a deployed configuration, with a braided or woven body that is gathered and coupled at a proximal end extending from an elongate proximal body 50. An inner deployment member 16 is coupled to a collet 51 that also couples to a gathered distal end of the braided or woven body 12. In this example, the collet is also connected to the central electrically conductive region 40. FIGS. 3B and 3C show a similar example of an electrical applicator, shown deployed with the braided or woven body formed into a funnel having a distal-facing outer region. In FIGS. 3B-3C, the central electrode (central electrically conductive region 40) is open, which may form a passage or channel through which another device or material (e.g., fluid) may be applied.

In FIGS. 1A-1B, 2A-2B and 3A-3C, the electrical applicator devices each include a braided electrode that may be constructed using a conductive wire, such as a NiTi wire, stainless steel, etc. For example, an electrically insulated NiTi wire or other electrically conductive material may be used for some or all of the strands forming the braided or woven body. The braided or woven body can be constructed over a metal mandrel of desired diameter and shape-set post braiding in order to set the desired outer diameter of the braided or woven body. The braided or woven body may be formed into an electrode assembly as shown, and the braided or woven body can be coupled onto a distal end of an elongate member (e.g., elongate proximal body, in some examples, a catheter), or may be used as a part of device that does not include an elongate member. The invertible electrode may be formed into the deployed configuration by pulling a pull mechanism (e.g., an inner deployment member, such as a rod, wire, catheter, microcatheter, etc.) that may be attached to the distal end of the braided or woven body. When the pull mechanism is activated, the distal end of the braided or woven body is pulled proximally causing it to invert inside on itself and forming the funnel shaped profile, which may have a distal-facing end (or distal-facing edge) on which the one or more electrically conductive regions (electrodes) may be formed. A center electrode may also be included and as described above, may be configured to be located approximately on the same plane as the largest diameter of the formed funnel.

The braided or woven body of FIG. 1A may be considered partially deployed because the body may be initially held within an outer cannula (not shown) that constrains the expansion of the braided or woven body so that the entire braided or woven body has a small diameter. When the braided or woven body is extended, e.g., by pushing the inner deployment member in variations in which the inner deployment member is fixed to the distal, gathered end of the braided or woven body, and/or by pushing distally from the proximal gathered end of the braided or woven body, the braided or woven body may self-expand into the shape shown in FIG. 1A. The device may be further deployed as described above, by pulling the inner deployment member proximally to form the funnel shape, as shown in FIG. 1B.

When the funnel-shaped electrical applicator is deployed within the body near the target region, it may be pushed against the tissue to be treated and electrical energy, such as (but not limited to) nanosecond pulsed electrical energy may be applied. For example, the devices described herein may be configured to deliver energy to a cardiac tissue; when inverted, the distal-facing end of the device including the one or more braided electrode(s) may be pressed against a cardiac tissue, along with the central electrode, and energy may be applied. In case of monopolar operation, the center electrode may not have to be used, or may be electrically coupled to the electrode(s) on the braided or woven body. In case of bipolar configuration, as shown in FIG. 3A, both the center electrode and the outer electrode(s) on the funnel shape formed by the braided or woven body are in contact with the tissue.

FIGS. 4A and 4B illustrate examples of tissue treated using an electrical applicator similar to that described herein in a monopolar configuration. In FIG. 4A an electrode applicator was used in a monopolar configuration so that the central electrode and a single, circumferential electrode formed on the distal face of inverted braided or woven body were electrically coupled together and to a pulse generator; a separate return path (e.g., ground pad) may be used. In FIG. 4A, energy from the pulse generator was applied to the test tissue (a cut potato) at 5 kV, using 200 ns pulses at 8 Hz; 100 pulses were applied. The image was taken 23 hours after the energy was applied and shows three regions 401 of treatment. Similar results were seen using animal tissue, as shown in FIG. 4B. In FIG. 4B, the image was taken between 4-4.5 hours after the application of monopolar nanosecond pulses applied as described above, using 5 kV or 7 kV, for 100 pulses at 200 ns/pulse at 6 Hz.

FIGS. 5A and 5B shows results with an electrical applicator as described herein in a bipolar configuration. In this example, the central electrode and a single, circumferential electrode formed on the distal face of inverted braided or woven body were each electrically coupled to a pulse generator to apply bipolar nanosecond pulsed electrical energy. In FIG. 5A, energy from the pulse generator was applied to the test tissue (a cut potato) at 12 kV, using 200 ns pulses at 8 Hz for 100 pulses. The image was taken 23 hours after the energy was applied. Three regions 501 of treatment are shown. Similar results were seen using animal tissue, as shown in FIG. 5B, which was taken 4.5-5 hours after the application of the bipolar nanosecond pulsed stimulation, using 10 kV or 12 kV, for 100 pulses at 200 ns/pulse at 6 Hz. In this example, the bipolar configuration resulted in a fairly uniform distribution of the energy to the tissue.

The invertible braided electrode designs described herein may generally be used as a distal part of the apparatus, e.g., electrode applicator for treatment of tissue, such as for treatment of atrial fibrillation, ventricular tachycardia, other cardiac related ablations. These electrode applicators may also be used to apply nanosecond pulsed electrical fields to other parts of the body. Alternatively or additionally, these electrical applicators may be used to apply other types of electrical energy, including, e.g., radio-frequency (RF) and/or micro-puled based fields. As mentioned, these invertible electrical applicators may be part of a catheter device, scope device, laparoscope device, endoscope device, etc. (e.g., may include an elongate proximal body extending proximally from the funnel shaped distal end). Such devices may be used, e.g., during minimally invasive procedures, and/or as a part of a surgery, such as (but not limited to) a cardiac surgery. In particular, since these devices may expand from a relatively narrow diameter within, for example, a lumen of a catheter, into a much larger diameter including the electrodes, they may be used in a procedure in which it may be desirable to treat (e.g., ablate) region of tissue.

The distance between outer and inner electrodes (e.g., the center electrode and one or more electrodes formed at least partially around the distal-facing outer region of the electrode) may be varied. Thus, the same applied voltage may vary the strength of the applied electrical field.

Any of the electrical applicator devices described herein may be configured so that the distal end is steerable. Thus, the distal, funnel shaped region may be steerable. The distal end region may be steerable by manipulating one or more tendons, which may be pulled and/or pushed from a proximal end region of the device. In some variations the distal end region (e.g., the funnel-shaped region) may be coupled to one or more tendons and used to pull and/or push proximally to steer the distal end region. For example, FIG. 6A shows one example of an electrical applicator device that may be configured as any of the devices described herein, such as FIGS. 1A-1B, 2A-2B, 3A-3C, 8A-8B, 9A-9B, 10A-10D, 11A-11B, 12A-12B, etc. In FIG. 6A, the device 610 includes a woven body 12 that forms a funnel shape 32 at the distal end when the woven body inverts onto itself during deployment. A central electrode 40 may be at least partially surrounded by the one or more electrode(s) 30 on the periphery of the funnel shape (e.g., on the distal end region 34). A bending region 56 may be positioned at or near the interface between the elongate body 50 and the funnel 32. The bending region 56 may include one or more pivoting region, such as vertebra, regions of variable stiffness, or the like that may provide predictable bending 622 in one or more directions. As mentioned, the device may include one or more (e.g., two or more, three or more, etc.) pull wires or tendons (not shown in FIG. 6A-6B) that may be attached to the funnel-shaped portion to provide steering by causing the bending region 56 to bend. In FIG. 6B the distal end region of the device, including the funnel shape 32, is shown deflected downwards.

FIG. 6C illustrates one example of an electrical applicator such as the one shown in FIGS. 6A-6B bending between about 10 degrees and about 270 degrees at a bending region 56. In FIG. 6A an inner deployment member (not visible) is pulled proximally so that the center electrode 40 is surrounded by the funnel shape 32 including one or more electrodes. The bend region 56 may include a hinging region (e.g., a region of different stiffness, etc.) so that pulling on a pull wire or tendon (not shown) may deflect the distal end to bend. The amount of the bend may depend on the amount that the pull wire or tendon is displaced. Thus, the device may be steered within the body.

An electrical applicator device such as the one shown in FIGS. 1A-1B and 2A-2B was also tested on a tissue model (e.g., potato) and the results are shown in FIGS. 7A-7B. In FIG. 7A, lesions of the test tissue were made by the application of bipolar nanosecond pulsed energy as described above. The images in FIGS. 7A and 7B were taken 24 hours after the application of the energy. As shown, relatively large (e.g., 12 mm diameter) and uniform lesions were formed by the application of energy. As shown in FIG. 7B, which illustrates the depth of penetration (in FIG. 7B, the tissue model has been sectioned through a lesion and the two cut pieces of model tissue arranged side-by-side). As can be seen, the depth of penetration was approximately 5.5 mm into the model tissue.

In any of these devices, the center electrode (the second conductive region) may be formed of a solid electrode (as shown in FIGS. 1A-1B, 2A-2B, and 3A-3C), or it may be formed of a braided or woven material, similar or identical to the electrodes formed on the funnel-shaped body. For example, FIGS. 8A and 8B illustrate one example of an electrical applicator (e.g., an invertible braided electrode) in which both the funnel-shaped body including the one or more electrodes and the central electrode is formed of a braided or woven material (e.g., wires). The two may be formed of the same braided or woven body or two separated braided or woven regions. In FIG. 8A, the electrical applicator 810 includes a braided or woven body 812 that extends proximally to distally and is gathered at the proximal end and coupled to an elongate proximal body (e.g., catheter) 850. A region of the braided or woven body may be configured to form the distal end region 834 of the distal-facing funnel shape when the device is deployed. The one or more electrodes on the braided or woven funnel body may be formed, as described above, on or from the portion of the braided or woven body that becomes the outer edge of the distal-facing funnel shape when the device is inverted. For example, the one or more electrodes on or around the distal-facing outer region may be formed by removing insulation from electrically conducting wires forming the braided or woven body.

The distal end of the braided or woven body in this example is coupled to a collet or ring 826 to which a second braided or woven body 840 is coupled at one, gathered, proximal end. The distal end of this second braided or woven body 840 is coupled to an inner puller that may be configured as the inner deployment member 816. Pulling the inner deployment member 816 may compress the second braided or woven body 840 (into a center electrode 840') and may invert the first braided or woven body 812, to form the funnel-shaped body wherein the second braided or woven body 840 acts as a flattened center electrode 840', as shown in FIG. 8B. In this example, the center electrode may be positioned within the distal-facing opening 830 of the funnel-shaped and may potentially expand to a larger diameter than would be possible with a convention solid electrode, allowing the undeployed configuration to be compact, e.g., for transport through a catheter. In some examples the first braided or woven body and the second braided or woven body may be portions of the same braided or woven body. In some examples a separate inner deployment member may be used to invert the first braided or woven body to form the funnel shape and/or to flatten the second braided or woven body forming the center electrode.

Any of the apparatuses described herein (e.g., any of the electrical applicators or systems including them) may be configured to have more than one distal-facing funnel, which may be concentrically arranged, in addition to or instead of a central electrode. For example, FIGS. 9A-9B, 10A-10D and 11A-11B illustrate examples of electrical applicator devices that include a pair of concentrically arranged funnels each including one or more electrodes on the distal-facing outer regions. FIG. 9A shows a schematic of one example of a device including a braided or woven body/bodies that are configured to form a first funnel shape from a first (proximal) portion of the braided or woven body and a second funnel shape (e.g., from a second distal portion of the braided or woven body). The first funnel shape may be arranged concentrically around the second funnel shape. In FIG. 9A, the device is shown not fully deployed. The device includes a first braided or woven body 966 and a second braided or woven body 968 that are separated by a neck member 933. An inner deployment member 916 may be coupled to the end of the second braided or woven body 968 and may be slidably movable within the neck member 933; pulling the inner deployment member 916 proximally may invert both the first braided or woven body to form the first (outer) funnel shape and the second braided or woven body to form the second (inner) funnel shape. Alternatively, in some examples, the first braided or woven body and the second braided or woven body may be separately deployed by each coupling to separate inner deployment members that may be arranged telescopically (e.g., concentrically) or in some examples, side-by-side, and coupled to the distal end of the first braided or woven body and the second braided or woven body, respectively. FIG. 9B shows a side view of the same device as shown in FIG. 9A. Either device may optionally include a center electrode 940. The first woven body and the second woven body may each include a circumferential region 924, 924' (or 925) that buckles or bends to form the distal-facing end of the funnel shape when deployed. One or more electrodes may be positioned on or integrated into each of these circumferential regions so that the one or more electrodes are present on each distal-facing end, as described herein. The center electrode may be electrically insulated from the one or more electrodes on the braided or woven bodies 966, 968.

FIGS. 10A-10B illustrate an example of electrical applicators having a pair of concentric funnels (similar to that shown undeployed in FIGS. 9A-9B) in the deployed configuration. Each funnel may include one or more electrodes arranged at least partially around the distal-facing outer regions (e.g., distal-facing edge and/or outer and/or inner edge region) of the funnel. An optional center electrode 1040 may also be included. The center electrode may be positioned at the end of an inner puller 1016 that couples to the distal end 1026 of the mesh forming the inner funnel. In FIG. 10A, a first electrode 1030 is included on the distal-facing outer region of the first funnel 1033 and may be on the inner face of the funnel; a second electrode 1030' is included on the distal-facing outer region of the second funnel 1033' and may be on the outer face of this portion of the funnel, as shown. This may allow the device, when operating in a bipolar configuration, to apply energy between the funnels at the distal end opening, which may apply energy to tissue from the electrode(s) on one funnel to the electrode(s) on the other funnel. In FIG. 10A the device may be used, for example, as part of an antrum ablation procedure. The device may circumferentially ablate tissue 1080, 1082 between the two funnel shaped bodies. FIG. 10B shows another example of an electrical applicator device in which the shapes of the inner and outer funnels may be different than those shown in FIG. 10A. FIG. 10B illustrates a method of ablating an antrum-ostium of a vessel 1085. The device may be similar to that shown in FIGS. 9A-9B. In operation, the device may be used as part of a catheter procedure, for delivery in an un-deployed configuration within a body lumen, such as a vessel. Once near the target region of the tissue to be treated (e.g., ablated), the device may be extended distally and deployed, e.g., by pulling an inner deployment member proximally (and/or by advancing the proximal end of the first braided or woven body distally), as described, above. This may form the inner and outer funnel members, as shown. As previously stated, the funnel shape may be formed without inverting when deploying the device comprises, for example, simply allowing a linear device to expand and form a funnel shape configuration.

In some methods, the apparatuses described herein may be used to detect or sense electrical signals from the tissue either before or during operation, including before or during an ablation. For example, these apparatuses (in either inverted or linear configuration) may be used to pace the heart. This can be performed, for example, across pairs of electrodes on the catheter itself, or from the catheter to a separate electrode, e.g., on a distal element such as a catheter, e.g., a coronary sinus (CS) catheter. Thus, any of these apparatuses can be used for mapping with or without ablation, of heart tissue. For example, the one or more electrodes on the braided or woven body may be used for mapping either with the central electrode or with an electrode on a separate device, e.g., catheter. Near and/or far field electrogram signals can be sensed with the device. In some methods the device may be used to sense electrical activity (e.g., electrogram) from the tissue to determine a region to ablate or to further ablate.

Any of the apparatuses described herein may also include a centering guide (centering feature) to assist in positioning the apparatus within the tissue. Thus any of these apparatuses may include a centering guide to assist in positioning the apparatus so that the electrodes are oriented relative to the tissue. In some examples the apparatus may include a centering guide to position the electrodes of the apparatus relative to the walls of a vessel or chamber, for example, relative to an antrum/ostium region of a vessel, such as the heart's pulmonary veins, enabling proper positioning and more efficient ablation while achieving PVI (Pulmonary Vein Isolation). In some implementations navigating the apparatuses (e.g., towards the pulmonary veins) and proper positioning may be challenging, and if in certain situations the apparatus is placed off-center (e.g., to the antrum of the PV or other target location), desired ablation may not be achieved. The centering guide may be expandable and/or collapsible. Further, the centering guide may be configured to be located proximal to the distal end region (including the funnel) and/or may be positioned distally from the distal end region and funnel.

For example, FIGS. 10C and 10D illustrate examples of electrical applicators having a centering guide 1095 that extends distally from the concentric funnels 1033, 1033' to assist in positioning the apparatus so that the electrodes 1030, 1030' are oriented relative to the tissue, and/or may assist in holding the electrodes against the tissue. In FIGS. 10C and 10D, the centering guide is shown as an inflatable member that is expanded into the lumen of an antrum-ostium of a vessel 1085. The centering guide extends from the distal end of the inner puller 1016.

Any of the apparatuses described herein may include a centering guide. For example, the centering guide may be on a distal end (e.g., of the inner deployment member) or on a proximal end, proximal to the funnel, e.g., on a distal end region of the deployment member, such as cannula. The centering guide may be expandable (e.g., balloon, basket, splines, ribs, etc.). In some examples the centering guide is inflatable. In some examples the centering guide is a basket (kitted, woven, braided, etc.) that expands and/or collapses released or captured by the cannula, or by connection to one or more tendons (e.g., pull/push wires, etc.). Thus, any of the apparatuses described herein may include one or more additional centering guides that can be a part of the apparatus or an additional device that can be used in conjunction with the apparatus to enable the centering with regard to the anatomical structure into which the treatment is to be applied, such as (but not limited to) a lumen of a vessel. In general, the centering guide may be an expandable, atraumatic projection that may extend distally from or around a distal end region of the apparatus. The centering guide may alternatively be configured to temporarily anchor the device in position within the body (e.g., body lumen). Thus, any of these centering guides may also be referred to as anchors. Any of these apparatuses may also or additionally use a guidewire for positioning the apparatus. For example, a balloon or splines may have integrated guidewire or a lumen for a guidewire that can be used to introduce the apparatus. In some examples a centering guide may also act as, or may include, an electrode for the application of pulsed energy to the tissue.

As mentioned above, the diameters of the funnel portion (and therefore the electrode size and shape) may be adjustable. FIGS. 11A-11B illustrates one example of an electrical applicator device having an adjustable outer diameter. In FIG. 11A the braided electrode device may be similar to that shown in FIGS. 1A-1B, 2A-2B, 3A-3C or 8A-8B, above. In this example the proximal end of the braided or woven body 1124 may be held within an outer member, such as catheter 1150. In FIG. 11A the device is shown with the funnel deployed and the proximal end of the braided or woven body, which may be coupled to an elongate body (e.g., catheter) extending proximally from the funnel, extending out of the outer catheter 1150. In this configuration the funnel shape has a first diameter, d₁, 1139. This diameter may be reduced by pulling the proximal end of the funnel shape (e.g., the proximal end of the braided or woven body 1124) into the outer catheter 1150, as shown in FIG. 11B. In FIG. 11B, the outer diameter of the funnel has been reduced to d₂ 1141.

In any of the apparatuses described herein, the center electrode may be longitudinally adjustable. For example, when applying energy from the electrical applicator device to a tissue surface, it may be desired to have the center electrode in the same plane (e.g., coplanar) with the one or more electrodes on the distal-facing outer region of the funnel shape. In examples such as those shown in FIGS. 11A-11B, in which the distal-facing opening of the funnel shape has an adjustable diameter, the distal-most edge of the funnel shape may vary as the funnel shape diameter changes. Thus, it may be beneficial to provide variations in which the longitudinal position of the center electrode may be adjusted.

FIGS. 12A-12B illustrate one example in which the center electrode (or center electrodes) is adjustable by including a positioning member extending proximally from the center electrode, within the braided or woven body, which is configured to allow the longitudinal position of the second conductive region to be adjusted relative to the distal-facing outer region of the funnel shape. This positioning member may extend within the inner deployment member or adjacent to the inner deployment member (in devices including an inner deployment member). The positioning member may be an elongate structure having a sufficient column strength to push the electrode, e.g., a rod, wire, cannula, microcannula, etc.

In FIG. 12A, the electrical applicator 1210 includes a braided or woven body 1224 that is shown in an un-deployed state. The device may be extended from an outer deployment cannula 1222. An inner deployment member 1216 may be attached to the distal end of the braided or woven body; in FIG. 12A a tubular or ring stop structure (e.g., collet 1226) is coupled to both the distal end of the braided or woven body and the inner deployment member. A positioning member (shown as rod 1205) may be coupled to the center electrode 1240 and may be proximally adjusted by moving distally-to-proximally 1209 (as shown by arrows in FIG. 12B) on the device in order to adjust the position of the center electrode. The positioning member may pass through the collet 1226. In some variations the positioning member may also be the same as the inner deployment member, which may be pulled back to deploy the device into the funnel shape, and the funnel shape may be retained even as the inner deployment member is advanced distally to position the center electrode. As described above, the device may be configured to preferentially bend or buckle (e.g., the braided or woven body) at a midline region of the braided or woven body to form a funnel having a distal-facing outer end; one or more electrodes 1230 may be arranged on or around this distal-facing outer end. In this example, the tubular ring or stop (e.g., collet) may be coupled to the distal end of the braided or woven body, but the inner deployment member may slide laterally (e.g., distally-to-proximally) within the ring or stop.

The structure of the center electrode can also be formed of the deployable structure, e.g., may also be made from the braided or woven material, such as NiTi braided wire, as described above; thus the diameter of the outer electrode may vary depending on the outer diameter of the NiTi braid.

An invertible braided electrode (e.g., an electrical applicator) as described herein can be used in any application for surface ablation. For example, these devices may be used for bipolar point-by-point ablation of even relatively large footprint ablation, such as for pulmonary vein isolation (PVI) in left atrium (LA) to treat atrial fibrillation (AF). To gain access to the LA of a heart, a puncture of the femoral vein may be performed using the needle under fluoroscopic and/or ultrasound guidance. After the puncture under fluoroscopic guidance a 0.032-inch J-tip guidewire is advanced. The needle may be removed, and a sheath introducer (usually 8-12 F) may be inserted into the vein and then flushed. The trans-septal sheath and dilator may be advanced over the guidewire to the superior vena cava (SVC). Once the sheath has reached 3 to 4 cm superior to the cavoatrial junction, the wire may be removed. The transseptal puncture needle may then be advanced under fluoroscopic guidance until it reaches the sheath tip. The needle is advanced with the stylet inserted until it reaches about 4 cm from the tip. The stylet may prevent the needle tip from scraping the inner lumen of the sheath. The stylet can then be removed. Puncture may be performed, and sheath advanced into the LA. The catheter with the braided invertible electrode may then be introduced in the LA through the sheath and the distal end of the catheter containing the center electrode is pulled back via mechanism in the catheter's handle. As a result of the described pullback, the braided or woven body may expand and then inverts inside on itself forming the funnel shape with a central electrode and one or more electrode on the distal-facing end of the outer region of the funnel shape that may be arranged co-planar to each other. The deployed electrical applicator may be used in a monopolar or bipolar configuration, as described above. As mentioned above, when the device is used in a monopolar configuration a neutral (e.g., ground or dispersive electrode, such as a ground pad) connected to the pulse generator may be placed in contact with the patient's body.

In some example, the apparatuses described herein may be deployed from a compressed configuration in which the funnel shape is already formed. For example, FIGS. 13A-13D illustrates one example of an electrical applicator having a funnel shape in which the braided or woven body 1324 is already formed into a funnel shape but is held within an outer (e.g., deployment) cannula 1322. In FIG. 13A the device may be pushed distally, e.g., from a proximal end of the device, to extend the device out of the outer cannula and/or the outer cannula may be pulled proximally (as shown in FIG. 13B) to extend the funnel shape out of the outer cannula, where it may self-expand into the funnel shape, as shown in FIGS. 13C1-13C2. All or a portion of this outer funnel shape may an electrically conductive region (e.g., electrode) 1330. For example, the distal-facing end region may be an electrode, or a portion of the annular distal-facing end region may be an electrode, as described above. The device may also include a central electrode 1340 within the funnel mouth, as shown in FIG. 13D.

In any of these electrical applicator apparatuses (e.g., devices) the inner deployment member may be configured to form a funnel shape having a distal-facing outer region in which a first electrically conductive region may be formed at least partially around the distal-facing outer region. The size (e.g., diameter) of the electrically conductive region on the distal-facing outer region may be adjustable, so that the treatment size (and/or shape) may be adjusted. The size may be controlled by controlling the expansion of the funnel shape. For example, in FIGS. 13A-13D the funnel shape 1324 may be generally configured to self-expand when released from the deployment cannula 1322, as shown in FIGS. 13C1 and 13C2. In FIG. 13C1 the cannula 1322 is just partially withdrawn proximally (arrow 1333), so that the funnel 1324 is partially expanded, e.g., from the collapsed diameter, d₁ (shown in FIG. 13B) to an intermediate diameter, d₂ (shown in FIG. 13C1). The relative position of the cannula and the funnel shape 1324 having a distal-facing outer region 1334 may be controlled to select the diameter of the distal-facing outer region 1334 and, therefore, the diameter and shape of the first electrically conductive region 1330 (first electrode) that is formed around the distal-facing outer region 1334. The first electrically conductive region 1330 may be configured to expand and contract as the distal-facing outer region 1334 expands and contracts. For example, the distal-facing outer region may be formed of multiple regions that are electrically connected (forming a single conductive region, e.g., electrode), as described herein, or may be formed of one or more electrically conductive elements that are flexible and/or are coupled, e.g., in a sigmoidal or zig-zag pattern around the distal-facing outer region, permitting expansion and contraction with the distal-facing outer region.

Moving the cannula 1322 further proximally may allow the funnel-shaped distal-facing outer region to further expand, to a maximum expanded diameter, d₃, as shown in FIG. 13C2. In this example, the first electrically conductive region 1330 (first electrode) that is formed around the distal-facing outer region 1334 of the funnel 1324 is fully expanded. The funnel, and therefore the distal-facing outer region and first electrode (electrically conductive region) is collapsible back to a smaller diameter (e.g. any dimeter between, and including, d₁ and d₃) by moving the cannula distally or the funnel may be enlarged, or allowed to expand, by withdrawing the cannula proximally. In this example, the second electrically conductive region 1340 is configured to be surrounded by the distal-facing outer region and therefore the first electrically conductive region 1330 for applying energy between the first electrode (electrically conductive region 1330) and the second electrode (second electrically conductive region 1340). In any of these examples, the second electrically conductive region may be coupled to an inner deployment member, so that the funnel shape may be initially formed by extending the distal end with the knitted, woven or braided region out of the delivery/deployment member or by withdrawing the delivery/deployment member proximally. The funnel shape may be collapsed into the deployment cannula 1322 as shown in FIGS. 13A-13B.

FIG. 14 shows another example of an electrical applicator device that is preformed into the funnel shape 1467 but may be compressed into a deployment configuration, as described above. This example also includes a braided or woven body 1424, and a distal-facing outer region having an edge 1434, along which one or more electrodes 1430 may be formed or coupled. The funnel shape may be formed by self-expansion after the device is moved distally relative to the outer cannula 1422; alternatively or additionally, the device may be deployed by pulling the distal end of the braided or woven body proximally. As mentioned above, multiple electrodes may be formed at the distal-facing outer region of the funnel shape by exposing uninsulated regions of some of the wires forming the braided or woven body at the distal facing outer region. The one or more electrically conductive wires forming each electrode region may form the electrode, and electrodes may be separated from each other by insulating regions. In some examples multiple conductive contacts, such as wires or other electrodes, may be positioned on the braided or woven body and each electrically connected to one or more conductive wires of the braided or woven body to form the individual electrodes.

Any of the electrode applicators described herein may also be configured to couple to a pulse generator for delivering high voltage, sub-nanosecond pulses into the target tissue. In particular, the electrical applicator (apparatuses, devices, or systems including them) may be configured to isolate the high voltage power applied by the pulse generator from the hands of the operator to prevent accidental harm to the operator. In some variations, the connection to the pulse generator may be an electrical cable that is connected to the electrical applicator distally from a handle. In some variations the electrical cable connection to the pulse generator may be connected to the handle or proximally to the handle. In some variations the electrical applicator may not include a handle.

Any of the apparatuses described herein may be configured to deliver a fluid, such as a conductive solution (e.g., saline) to the electrode region(s), and in particular, between the first electrically conductive region and the second electrically conductive region (or additional electrically conductive regions. The addition of saline in these regions may help prevent arcing. For example, when applying the applicator device against a tissue, such as against a relatively flat region of a tissue (e.g., on a surface of an abdominal cavity), a conductive solution, such as saline, may be applied from the distal end of the device during the application of electrical treatment, such as sub-microsecond pulsing, as described herein. Any of the expandable funnels described herein may include a coating or layer to prevent or limit fluid (e.g., saline, blood, etc.) from passing through the funnel region. Thus, in some examples the use of a fluid-impermeable barrier added to the funnel to limit or prevent fluid from escaping through the funnel may maintain a local region of solution having known conductive properties, such as saline. For example, any of these apparatuses may include a lining on the mesh or material forming the funnel region to make it relatively saline-tight.

For example, returning now to FIGS. 11A and 11B showing an apparatus forming a distal-facing funnel 1124 when the inner member 1151 is pulled proximally, in some examples the inner member 1151 may also include one or more ports for delivery of fluid (e.g., saline, etc.). Also, the funnel-shaped (or conical) distal end may form a fluid-impermeable barrier for holding the saline adjacent to the electrodes (first electrode and second electrode).

As mentioned, any of these apparatuses may be configured to be part of and/or used at the distal end of a catheter or cannula. Alternatively, in any of these examples, the features described herein for catheters or cannulas may likewise be applied to scopes (e.g., endoscopes, laparoscopes, etc.) depending on the type of deployment used in a particular example or implementation.

FIG. 15 illustrates one example of a system 100 (also referred to herein as a high voltage system or a sub-microsecond generation system) for delivering high voltage, fast pulses of electrical energy that may include an electrical applicator device 102, a pulse generator 107, footswitch 103, and user interface 104. Footswitch 103 is connected to housing 105 (which may enclose the electronic components) through a cable and connector 106. The elongate applicator device (e.g., catheter) 102 may include electrodes and is connected to housing 105 and the electronic components therein through a cable 137 and high voltage connector 112. The high voltage system 100 may also include a handle 110 and storage drawer 108. The system 100 may also include a holder (e.g., holster, carrier, etc.) (not shown) which may be configured to hold the elongate electrical applicator device 102. The system may be configured for monopolar operation of the electrical applicator device and may optionally include a ground pad 152 or other neutral (dispersive) electrode to be placed in electrical communication with the patient to whom treatment is to be delivered. The return electrode may be coupled to the system by a cable 157 of any appropriate length, which may plug into the system.

In some cases, the applicator catheter includes imaging, such as one or more cameras and/or fiber optics at or near the distal end of the applicator catheter. The camera(s) may be forward-facing and/or side facing. The system 100 may be configured to display images (in real time, and/or recorded) taken by the applicator catheter, in order to identify the target region(s).

A human operator may select a number of pulses, amplitude, pulse duration, and frequency information, for example by inputting such parameters into a numeric keypad or a touch screen of interface 104. In some embodiments, the pulse width can be varied. A controller may send signals to pulse control elements within system 100. In some embodiments, fiber optic cables are used which allow control signaling while also electrically isolating the contents of the metal cabinet with sub-microsecond pulse generation system 100, e.g., the high voltage circuit, from the outside. In order to further electrically isolate the system, system 100 may be battery powered instead of being powered from a wall outlet.

As mentioned above, any of the apparatuses described herein may be implemented in robotic systems that may be used to position and/or control the electrodes during a treatment. For example, a robotic system may include a movable (robotic) arm to which elongate applicator tool is coupled. Various motors and other movement devices may be incorporated to enable fine movements of an operating tip of the elongate applicator tool in multiple directions. The robotic system and/or elongate applicator tool may further include at least one image acquisition device (and preferably two for stereo vision, or more) which may be mounted in a fixed position or coupled (directly or indirectly) to a robotic arm or other controllable motion device. In some embodiments, the image acquisition device(s) may be incorporated into the elongate applicator tool.

Any appropriate nanosecond scale pulsing may be applied. For example, the pulse profiles may also include a rise and/or fall times for pulses that may be less than 20 ns, about 20 ns, about 25 ns, about 30 ns, about 40 ns, about 50 ns, about 60 ns, about 75 ns, or greater than 75 ns. In some embodiments, the pulse voltage may be less than 5 kV, about 5 kV, about 10 kV, about 15 kV, about 20 kV, about 25 kV, about 30 kV, or greater than 30 kV. In some embodiments, the current may be less than 10 A, about 10 A, about 25 A, about 40 A, about 50 A, about 60 A, about 75 A, about 100 A, about 125 A, about 150 A, about 175 A, about 200 A, or more than 200 A. In some embodiments, the pulse duration may be less than 10 ns, about 10 ns, about 15 ns, about 20 ns, about 25 ns, about 30 ns, about 40 ns, about 50 ns, about 60 ns, about 75 ns, about 100 ns, about 125 ns, about 150 ns, about 175 ns, about 200 ns, about 300 ns, about 400 ns, about 500 ns, about 750 ns, about 1 µs, about 2 µs, about 3 µs, about 4 µs, about 5 µs, or greater than 5 µs. The apparatuses (e.g., systems) described herein may include, in addition to the instrument (e.g., the elongate applicator tool), a pulse generator such as the one shown schematically in FIG. 15, configured to emit pulses in the sub-microsecond range.

### METHODS OF USING DEVICES

Any of the apparatuses (e.g., devices, etc.) may be used to treat a patient tissue, including, but not limited to methods and applications for using systems and/or devices such as those described herein for treating an anatomical structure or tissue, such as a wall, lumen, passage, cavity or vessel of any tissue, such as, e.g., a vein, artery, vessel, heart, trachea, pharynx, larynx, bronchi, ureter, urethra, fallopian tubes, cervix, uterus, intestine (large and/or small), pancreas and pancreatic duct, liver and hepatic ducts, rectum, esophagus, stomach, nasal cavity, seminal vesicles, bronchi, etc. Systems, devices and methodology disclosed herein are especially useful with non-thermal pulsed electric fields (e.g., nanosecond pulsed electric fields, etc.), however, in some implementations they can be also used with other various energy modalities, including (but not limited to) radiofrequency (RF). While devices and systems of the present disclosure find use in tubular anatomical structures, these device, system and methodologies can be used to apply treatment to non-tubular structures and surfaces, including those that are substantially flat as well. For example, such devices may be introduced from a smaller body lumen into a larger cavity and then expanded, for example, to apply energy to a flat non-tubular surface using only some of the electrodes on the expandable member. An anatomical cavity, tube, lumen, passageways or vessels will be referred here as a body lumen or a lumen. In some examples the body lumens may include various blood vessels (e.g., pulmonary veins) and device and methods of the present disclosure may be useful for treatment of various cardiac conditions, such as atrial fibrillation among others. As another non-limiting example, device and methods of the present disclosure may be used for treatment of endometriosis or conditions of the vocal folds. Electrodes for delivery of the treatment may include one or more electrode assemblies affixed, for example, to the surface of an expandable structure (for example, those shown in the examples of FIGS. 1-14) and configured to deploy from a catheter or other deployment member and conform, for example, to a portion of a wall of a body lumen.

In general, the apparatuses described herein may be used to treat a tissue by deploying the apparatus to the target tissue to be treated, which may include transforming the device into the funnel-shaped configuration, positioning it at or near the target tissue (e.g., against the wall, *en face* or circumferentially, to be treated) and applying energy through the device, e.g., between the first electrically conductive region (e.g., around all or a portion of the distal-facing funnel), and the second electrically conducive region, which may be within the funnel shape. For example, FIG. 16 illustrates one example of a method of treating a target tissue. In some implementations, the device may be deployed, optionally, by, e.g., withdrawing an inner deployment member of an electrical applicator proximally to invert a distal end region of a braided or woven body of the electrical applicator to form a funnel shape having a distal-facing outer region (step 1601). In other implementations, the device may be deployed in step 1601 without inverting the distal end region of a braided or woven body, for example, by allowing a self-expansion of the device into a funnel-shaped configuration. Once deployed, the funnel-shaped distal end region may be placed against the target tissue, or near the target tissue. For example, the first electrically conductive region on the distal-facing outer region of the apparatus may be positioned against a target tissue so that a second electrically conductive region that is surrounded by the distal-facing outer region within the funnel shape also contacts the target tissue (step 1603). Energy may then be applied from the apparatus (e.g., electrical energy, including but not limited to electrical energy within the sub-microsecond range, e.g., nanosecond range,), for example, applying a plurality of electrical pulses having an amplitude of at least 0.1 kV and a duration of less than 1000 nanoseconds to the target tissue from the first and second electrically conductive regions (step 1605).

Any of the apparatuses described herein may be used to treat the pulmonary system (e.g., lungs, pulmonary artery, etc.) by inserting the device, in an undeployed configuration, within the pulmonary system or portion thereof, and then deploying the device, e.g., to treat the pulmonary tissue. In some examples, as mentioned above, the method may include treating hepatic (e.g., liver) tissue, including inserting the apparatus to contact the target region of the liver tissue. In general, these methods may be used to treat "flat" tissues, or tissues that are not confined within a tubular structure.

Any of these apparatuses may be used for cardiac treatments. When used for cardiac use, the device may be used in the heart (e.g., within a chamber of the heart, such as the ventricle, atrium, etc.) and/or within the vessels and channels being monitored.

The apparatuses described herein may be used to treat endometriosis. For example, a doctor (e.g., surgeon) may make a tiny incision near the patient's navel and insert a slender viewing instrument (laparoscope), looking for signs of endometrial tissue outside the uterus. Thus, the devices described herein may be configured for working with a laparoscope or for use as part of a laparoscope. These apparatuses can provide information about the location, extent and size of endometriosis or information about a device to treat it. The apparatus may also be used to remove tissue, including tissue, affected by endometriosis by applying energy to ablate, including preferentially ablate, such tissue. In some examples the woven body described herein may be coupled to the end (distal end region) of a rigid or semi-rigid scope (e.g., endoscope, laparoscope, etc.). Similarly, these methods and apparatuses may be used to treat vocal folds and vocal cords (e.g., vocal polyps, etc.), including ablating/removing tissue on the vocal folds/cords or modifying the vocal folds/cords.

In general, the examples and methods described herein may include treatment of a subject (e.g., human or animal patient) by the application of therapeutic energy, including but not limited to short, high field strength electric pulses, while minimizing or avoiding the risk of harming non-target tissue. These examples and methods may be used for minimally invasive procedures, and may be particularly well suited, for example, for treatments of various conditions, disorders and diseases, such as, but not limited to benign, cancerous or precancerous tumors, lesions, and other types of abnormal tissue growth, and the like. These examples and methods may be also particularly well suited for use with various fully and partially automated systems, such as robotic systems. The apparatuses described herein may be configured as apparatuses (e.g., catheter apparatuses, cannula or scope-based apparatuses, other minimally-invasive devices and control systems, etc.) that can be used with a variety of different energy generating systems. Examples of cancers that may be treated include, but are not limited to: lung cancer, kidney cancer (e.g., kidney carcinoma), pancreatic cancer (e.g. pancreatic carcinoma), colon cancer (e.g., colon carcinoma), breast cancer, prostate cancer, etc. Examples of tumors that may be treated with the devices and methods of the present disclosure include a benign prostatic hyperplasia (BPH), uterine fibroid, and tissue associated with Barrett's esophagus. Any of these methods and apparatuses may alternatively or additionally be used percutaneously to insert the apparatus through a cut into the skin.

In general, the apparatuses described herein may be used to apply treatment to or through a lumen (e.g., endoluminally). For example, a prostate tumor (e.g., cancer) may be treated by advancing a device (e.g., flexible catheter) having an electrical applicator (e.g., an expandable funnel having a distal-facing annular electrically active region) through the patient's urethra, transurethrally. Alternatively, as just mentioned, the apparatus may be inserted through an incision into the body and applied percutaneously to the tissue to be treated. Transurethral delivery may include insertion of the treatment device, configured to include the expandable funnel having a distal-facing annular electrical active region (or just an expandable backed with a peripheral electrode) at the end, or configured to extend from the end, of a catheter inserted through the penis, through the urethra and into the prostate, where energy, including in particular pulsed sub-microsecond (e.g., nanosecond) energy may be applied to the tumor tissue.

In general, the apparatuses of the present disclosure may comprise additional elements, such as power supplies, and/or a high voltage connector for safely connecting the elongate applicator tool device to a high voltage power source. As described above, these systems and devices are configured to apply high voltage, sub-microsecond pulsed electrical energy.

The electrical applicators described herein may be any appropriate length (e.g., between about 4 inches and about 200 inches, between about 4 inches and about 100 inches, between about 4 inches and 30 inches long, between about 6 inches and 50 inches long, between about 6 inches and 25 inches long, between about 7 inches and 24 inches long, etc.) and may have any appropriate outer diameter in the undeployed state and/or deployed state.

Embodiments of the methods of the present disclosure may be implemented using computer software, firmware or hardware. Various programming languages and operating systems may be used to implement the present disclosure. The program that runs the method and system may include a separate program code including a set of instructions for performing a desired operation or may include a plurality of modules that perform such sub-operations of an operation or may be part of a single module of a larger program providing the operation. The modular construction facilitates adding, deleting, updating and/or amending the modules therein and/or features within the modules.

In some embodiments, a user may select a particular method or embodiment of this application, and the processor will run a program or algorithm associated with the selected method. In certain embodiments, various types of position sensors may be used. For example, in certain embodiment, a non-optical encoder may be used where a voltage level or polarity may be adjusted as a function of encoder signal feedback to achieve a desired angle, speed, or force.

Certain embodiments may relate to a machine-readable medium (e.g., computer readable media) or computer program products that include program instructions and/or data (including data structures) for performing various computer-implemented operations. A machine-readable medium may be used to store software and data which causes the system to perform methods of the present disclosure. The above-mentioned machine-readable medium may include any suitable medium capable of storing and transmitting information in a form accessible by processing device, for example, a computer. Some examples of the machine-readable medium include, but not limited to, magnetic disc storage such as hard disks, floppy disks, magnetic tapes. It may also include a flash memory device, optical storage, random access memory, etc. The data and program instructions may also be embodied on a carrier wave or other transport medium. Examples of program instructions include both machine code, such as produced by a compiler, and files containing higher level code that may be executed using an interpreter.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to perform or control performing of any of the steps, including but not limited to: displaying, communicating with the user, analyzing, modifying parameters (including timing, frequency, intensity, etc.), determining, alerting, or the like. In some exemplary embodiments hardware may be used in combination with software instructions to implement the present disclosure.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "mounted", "connected", "attached" or "coupled" to another feature or element, it can be directly mounted, connected, attached or coupled to the other feature or element or intervening features or elements may be present. In contrast, when a feature or element is referred to as being "directly mounted", "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting. For example, as used herein, the singular forms "a", "an" and "the" are intended to include the plural forms as well, unless the context clearly indicates otherwise. As used herein, the term "and/or" includes any and all combinations of one or more of the associated listed items.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present apparatuses and methods.

The terms "comprises" and/or "comprising," when used in this specification (including the claims), specify the presence of stated features, steps, operations, elements, and/or components, but do not preclude the presence or addition of one or more other features, steps, operations, elements, components, and/or groups thereof. Unless the context requires otherwise, "comprise", and variations such as "comprises" and "comprising," means various components can be co-jointly employed in the methods and articles (e.g., compositions and apparatuses including device and methods). For example, the term "comprising" will be understood to imply the inclusion of any stated elements or steps but not the exclusion of any other elements or steps.

Any of the apparatuses and methods described herein may include all or a sub-set of the components and/or steps, and these components or steps may be either non-exclusive (e.g., may include additional components and/or steps) or in some variations may be exclusive, and therefore may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value unless the context indicates otherwise. For example, if the value "10" is disclosed, then "about 10" is also disclosed. Any numerical range recited herein is intended to include all sub-ranges subsumed therein. Although various illustrative embodiments are described above, any of a number of changes may be made to various embodiments without departing from the scope of the disclosure as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the apparatuses and methods as it is set forth in the claims.

This disclosure is intended to cover any and all adaptations or variations of various embodiments. The scope of the inventions is defined by the appended claims.

## Claims

1. An electrical applicator device (10, 102, 610, 810, 1210), the device comprising:
a braided or woven body (12) having a distal end (26) and a proximal end (14);
an inner deployment member (16, 816, 916, 1216) extending within the braided or woven body and coupled to a distal attachment site of the braided or woven body, wherein the inner deployment member is configured to be movable to invert a distal end region (34, 834) of the braided or woven body to form a funnel shape (32, 55) having a distal-facing outer region;
a first electrode (30, 1030, 1330) formed at least partially around the distal-facing outer region of the inverted funnel shape; and
a second electrode (40, 1030', 1340) configured to contact tissue to be ablated and positioned at least partially within an opening of the funnel shape when the braided or woven body is inverted .

2. The device of claim 1, wherein the first electrode (30, 1030, 1330) is configured to be positioned in contact with the tissue to be ablated when the braided or woven body is inverted.

3. The device of claims 1 or 2, wherein the electrical applicator device is configured as a bipolar electrical applicator, optionally wherein the second electrode is distal to the distal attachment site of the braided or woven body.

4. The device of any of claims 1-3, further comprising an elongate proximal body (50) extending proximally from the funnel shape and one or more electrical connectors at a proximal end region of the elongate proximal body in electrical communication with the first and second electrodes.

5. The device of any of claims 1-4, wherein the braided or woven body comprises a conductive wire, optionally wherein the first electrode is formed of an uninsulated portion of the conductive wire, further optionally wherein a portion of the braided or woven body proximal to the distal-facing outer region is electrically insulated.

6. The device of any of claims 1-5, wherein the second electrode comprises a solid material or body or comprises braided or woven material that is similar or different than a material of the braided or woven body.

7. The device of any of claims 1-6, wherein the second electrode is configured to radially expand and/or longitudinally flatten when the inner deployment member (16) or a separate inner deployment member is withdrawn proximally, or wherein the second electrode is configured to radially expand when the braided or woven body expands into the funnel shape.

8. The device of any of claims 1-7, further comprising a positioning member extending within the braided or woven body proximally from the second electrode and configured to allow an adjustment of a longitudinal position of the second electrode relative to the distal-facing outer region of the funnel shape.

9. The device of any of claims 1-8, wherein a size, outer diameter and/or shape of either one or both of the first electrode and the second electrode is adjustable, optionally wherein the device is further comprising a bending region (56) proximal to the funnel shape (32, 55) and configured to be bent to steer the funnel shape.

10. The device of any of claims 1-9, wherein the device is configured for point-by-point ablation, optionally wherein the first electrode and the second electrode are coplanar when deployed.

11. The device of any of claims 1-10, wherein the device is further configured to detect electrical signals from the tissue to determine a region to ablate or to further ablate.

12. The device of any of claims 1-11, wherein the second electrode is positioned at or on the inner deployment member, optionally wherein the inner deployment member is configured to engage a releasable stop to prevent the distal end region of the braided or woven body to invert.

13. The device of any of claims 1-7, comprising:
a second region of the braided or woven body forming or configured to form a second funnel shape (1033'), the second funnel shape having a second distal-facing outer region, optionally wherein the second electrode is formed at least partially around the second distal-facing outer region, optionally wherein one of the funnel shape or the second funnel shape is configured to concentrically surround the other.

14. The device of claim 13, the device configured to circumferentially ablate the tissue between the funnel shape and the second funnel shape, optionally wherein the inner deployment member is configured to be withdrawn proximally to invert the braided or woven body to form at least one of the funnel shape and the second funnel shape, or both the funnel shape and the second funnel shape.

15. The device of claim 13, wherein the first region of the braided or woven body is continuous with the second region of the braided or woven body and/or wherein the first electrode and the second electrode are electrically coupled, optionally wherein the device is further comprising a neck member on the braided or woven body between a first region of the braided or woven body and the second region of the braided or woven body.

16. A system (100) comprising:
an electrical applicator of any one of claims 1-15; and
a pulse generator (107) configured to generate a plurality of electrical pulses having amplitude of at least 0.1 kV and a duration of less than 1000 nanoseconds, the electrical applicator configured to electrically couple to the pulse generator.

## Patentansprüche

1. Elektrische Applikatorvorrichtung (10, 102, 610, 810, 1210), die Vorrichtung umfassend:
einen geflochtenen oder gewebten Körper (12), der ein distales Ende (26) und ein proximales Ende (14) aufweist;
ein inneres Einsatzelement (16, 816, 916, 1216), das sich innerhalb des geflochtenen oder gewebten Körpers erstreckt und mit einer distalen Befestigungsstelle des geflochtenen oder gewebten Körpers gekoppelt ist, wobei das innere Einsatzelement konfiguriert ist, um beweglich zu sein, um einen distalen Endbereich (34, 834) des geflochtenen oder gewebten Körpers zu invertieren, um eine Trichterform (32, 55), die einen distal gerichteten äußeren Bereich aufweist, auszubilden;
eine erste Elektrode (30, 1030, 1330), die mindestens teilweise um den distal gerichteten äußeren Bereich der invertierten Trichterform herum ausgebildet ist; und
eine zweite Elektrode (40, 1030', 1340), die konfiguriert ist, um mit dem zu abladierenden Gewebe in Kontakt zu kommen und mindestens teilweise innerhalb einer Öffnung der Trichterform positioniert ist, wenn der geflochtene oder gewebte Körper invertiert wird.

2. Vorrichtung nach Anspruch 1, wobei die erste Elektrode (30, 1030, 1330) konfiguriert ist, um in Kontakt mit dem zu abladierenden Gewebe positioniert zu werden, wenn der geflochtene oder gewebte Körper invertiert wird.

3. Vorrichtung nach Anspruch 1 oder 2, wobei die elektrische Applikatorvorrichtung als bipolarer elektrischer Applikator konfiguriert ist, optional wobei die zweite Elektrode distal zu der distalen Befestigungsstelle des geflochtenen oder gewebten Körpers ist.

4. Vorrichtung nach einem der Ansprüche 1 bis 3, ferner umfassend einen länglichen proximalen Körper (50), der sich von der Trichterform proximal erstreckt, und einen oder mehrere elektrische Verbinder an einem proximalen Endbereich des länglichen proximalen Körpers in elektrischer Verbindung mit der ersten und der zweiten Elektrode.

5. Vorrichtung nach einem der Ansprüche 1 bis 4, wobei der geflochtene oder gewebte Körper einen leitfähigen Draht umfasst, optional wobei die erste Elektrode aus einem nicht isolierten Abschnitt des leitfähigen Drahts ausgebildet ist, und ferner optional wobei ein Abschnitt des geflochtenen oder gewebten Körpers proximal zu dem distal gerichteten äußeren Bereich elektrisch isoliert ist.

6. Vorrichtung nach einem der Ansprüche 1 bis 5, wobei die zweite Elektrode ein festes Material oder einen festen Körper umfasst oder geflochtenes oder gewebtes Material umfasst, das dem Material des geflochtenen oder gewebten Körpers ähnlich ist oder sich davon unterscheidet.

7. Vorrichtung nach einem der Ansprüche 1 bis 6, wobei die zweite Elektrode konfiguriert ist, um sich radial auszudehnen und/oder in Längsrichtung abzuflachen, wenn das innere Einsatzelement (16) oder ein separates inneres Einsatzelement proximal zurückgezogen wird, oder wobei die zweite Elektrode konfiguriert ist, um sich radial auszudehnen, wenn sich der geflochtene oder gewebte Körper in die Trichterform ausdehnt.

8. Vorrichtung nach einem der Ansprüche 1 bis 7, ferner umfassend ein Positionierungselement, das sich innerhalb des geflochtenen oder gewebten Körpers proximal von der zweiten Elektrode erstreckt und konfiguriert ist, um eine Einstellung einer Längsposition der zweiten Elektrode relativ zu dem distal gerichteten äußeren Bereich der Trichterform ermöglicht.

9. Vorrichtung nach einem der Ansprüche 1 bis 8, wobei eine Größe, ein Außendurchmesser und/oder eine Form einer oder beider der ersten Elektrode und der zweiten Elektrode einstellbar ist, optional wobei die Vorrichtung ferner einen Biegebereich (56) proximal zu der Trichterform (32, 55) umfasst, und konfiguriert ist, um gebogen zu werden, um die Trichterform zu steuern.

10. Vorrichtung nach einem der Ansprüche 1 bis 9, wobei die Vorrichtung für eine Punkt-für-Punkt-Ablation konfiguriert ist, optional wobei die erste Elektrode und die zweite Elektrode koplanar sind, wenn sie eingesetzt sind.

11. Vorrichtung nach einem der Ansprüche 1 bis 10, wobei die Vorrichtung ferner konfiguriert ist, um elektrische Signale von dem Gewebe zu erfassen, um einen Bereich zu bestimmen, der abladiert werden soll oder weiter abladiert werden soll.

12. Vorrichtung nach einem der Ansprüche 1 bis 11, wobei die zweite Elektrode an oder auf dem inneren Einsatzelement positioniert ist, optional wobei das innere Einsatzelement konfiguriert ist, um mit einem lösbaren Anschlag in Eingriff zu kommen, um ein Invertieren des distalen Endbereichs des geflochtenen oder gewebten Körpers zu verhindern.

13. Vorrichtung nach einem der Ansprüche 1 bis 7, umfassend:
einen zweiten Bereich des geflochtenen oder gewebten Körpers, der eine zweite Trichterform (1033') ausbildet oder konfiguriert ist, um eine solche auszubilden, wobei die zweite Trichterform einen zweiten, distal gerichteten äußeren Bereich aufweist, optional wobei die zweite Elektrode mindestens teilweise um den zweiten, distal gerichteten äußeren Bereich herum ausgebildet ist, optional wobei eine der Trichterform oder die zweite Trichterform konfiguriert ist, um die andere konzentrisch zu umgeben.

14. Vorrichtung nach Anspruch 13, wobei die Vorrichtung konfiguriert ist, um das Gewebe zwischen der Trichterform und der zweiten Trichterform kreisförmig zu abladieren, optional wobei das innere Einsatzelement konfiguriert ist, um proximal zurückgezogen zu werden, um den geflochtenen oder gewebten Körper zu invertieren, um mindestens eine der Trichterform und der zweiten Trichterform oder sowohl die Trichterform als auch die zweite Trichterform auszubilden.

15. Vorrichtung nach Anspruch 13, wobei der erste Bereich des geflochtenen oder gewebten Körpers mit dem zweiten Bereich des geflochtenen oder gewebten Körpers kontinuierlich ist und/oder wobei die erste Elektrode und die zweite Elektrode elektrisch gekoppelt sind, optional wobei die Vorrichtung ferner ein Halselement auf dem geflochtenen oder gewebten Körper zwischen einem ersten Bereich des geflochtenen oder gewebten Körpers und dem zweiten Bereich des geflochtenen oder gewebten Körpers umfasst.

16. System (100), umfassend:
einen elektrischen Applikator nach einem der Ansprüche 1 bis 15; und
einen Impulsgenerator (107), der konfiguriert ist, um eine Vielzahl von elektrischen Impulsen, die eine Amplitude von mindestens 0,1 kV und einer Dauer von weniger als 1000 Nanosekunden aufweisen, zu erzeugen, wobei der elektrische Applikator konfiguriert ist, um mit dem Impulsgenerator elektrisch gekoppelt zu werden.

## Revendications

1. Dispositif applicateur électrique (10, 102, 610, 810, 1210), le dispositif comprenant :
un corps tressé ou tissé (12) ayant une extrémité distale (26) et une extrémité proximale (14) ;
un élément de déploiement interne (16, 816, 916, 1216) s'étendant au sein du corps tressé ou tissé et accouplé à un site d'attachement distal du corps tressé ou tissé, dans lequel l'élément de déploiement interne est conçu pour pouvoir être déplacé pour inverser une région d'extrémité distale (34, 834) du corps tressé ou tissé pour former une forme d'entonnoir (32, 55) ayant une région externe à orientation distale ;
une première électrode (30, 1030, 1330) formée au moins partiellement autour de la région externe à orientation distale de la forme d'entonnoir inversé ; et
une seconde électrode (40, 1030', 1340) conçue pour venir en contact avec un tissu devant subir une ablation et positionnée au moins partiellement au sein d'une ouverture de la forme d'entonnoir lorsque le corps tressé ou tissé est inversé.

2. Dispositif selon la revendication 1, dans lequel la première électrode (30, 1030, 1330) est conçue pour être positionnée en contact avec le tissu devant subir une ablation lorsque le corps tressé ou tissé est inversé.

3. Dispositif selon les revendications 1 ou 2, dans lequel le dispositif applicateur électrique est configuré en tant qu'applicateur électrique bipolaire facultativement dans lequel la seconde électrode est distale par rapport au site d'attachement distal du corps tressé ou tissé.

4. Dispositif selon l'une quelconque des revendications 1 à 3, comprenant en outre un corps proximal allongé (50) s'étendant proximalement par rapport à la forme d'entonnoir et un ou plusieurs connecteurs électriques au niveau d'une région d'extrémité proximale du corps proximal allongé en communication électrique avec les première et seconde électrodes.

5. Dispositif selon l'une quelconque des revendications 1 à 4, dans lequel le corps tressé ou tissé comprend un fil conducteur, facultativement dans lequel la première électrode est formée d'une partie non isolée du fil conducteur, facultativement en outre dans lequel une partie du corps tressé ou tissé proximale par rapport à la région externe à orientation distale est électriquement isolée.

6. Dispositif selon l'une quelconque des revendications 1 à 5, dans lequel la seconde électrode comprend un matériau ou corps plein ou comprend un matériau tressé ou tissé qui est similaire ou différent par rapport à un matériau du corps tressé ou tissé.

7. Dispositif selon l'une quelconque des revendications 1 à 6, dans lequel la seconde électrode est conçue pour s'expanser radialement et/ou s'aplatir longitudinalement lorsque l'élément de déploiement interne (16) ou un élément de déploiement interne séparé est retiré proximalement, ou dans lequel la seconde électrode est conçue pour s'expanser radialement lorsque le corps tressé ou tissé s'expanse en la forme d'entonnoir.

8. Dispositif selon l'une quelconque des revendications 1 à 7, comprenant en outre un élément de positionnement s'étendant au sein du corps tressé ou tissé proximalement par rapport à la seconde électrode et conçu pour permettre un ajustement d'une position longitudinale de la seconde électrode par rapport à la région externe à orientation distale de la forme d'entonnoir.

9. Dispositif selon l'une quelconque des revendications 1 à 8, dans lequel une taille, un diamètre externe et/ou une forme de soit l'une soit l'une et l'autre parmi la première électrode et la seconde électrode sont ajustables, facultativement dans lequel le dispositif comprend en outre une région de pliage (56) proximale par rapport à la forme d'entonnoir (32, 55) et conçue pour être pliée pour orienter la forme d'entonnoir.

10. Dispositif selon l'une quelconque des revendications 1 à 9, dans lequel le dispositif est conçu pour une ablation point par point, facultativement dans lequel la première électrode et la seconde électrode sont coplanaires lorsqu'elles sont déployées.

11. Dispositif selon l'une quelconque des revendications 1 à 10, dans lequel le dispositif est configuré en outre pour détecter des signaux électriques en provenance du tissu pour déterminer une région devant subir une ablation ou une ablation supplémentaire.

12. Dispositif selon l'une quelconque des revendications 1 à 11, dans lequel la seconde électrode est positionnée au niveau de, ou sur, l'élément de déploiement interne, facultativement dans lequel l'élément de déploiement interne est conçu pour venir en prise avec un arrêt libérable pour empêcher la région d'extrémité distale du corps tressé ou tissé de s'inverser.

13. Dispositif selon l'une quelconque des revendications 1 à 7, comprenant :
une seconde région du corps tressé ou tissé formant ou conçue pour former une seconde forme d'entonnoir (1033'), la seconde forme d'entonnoir ayant une seconde région externe à orientation distale, facultativement dans lequel la seconde électrode est formée au moins partiellement autour de la seconde région externe à orientation distale, facultativement dans lequel l'une parmi la forme d'entonnoir ou la seconde forme d'entonnoir est conçue pour entourer de manière concentrique l'autre.

14. Dispositif selon la revendication 13, le dispositif étant conçu pour effectuer une ablation circonférentielle du tissu entre la forme d'entonnoir et la seconde forme d'entonnoir, facultativement dans lequel l'élément de déploiement interne est conçu pour être retiré proximalement pour inverser le corps tressé ou tissé pour former au moins l'une parmi la forme d'entonnoir et la seconde forme d'entonnoir, ou à la fois la forme d'entonnoir et la seconde forme d'entonnoir.

15. Dispositif selon la revendication 13, dans lequel la première région du corps tressé ou tissé est continue avec la seconde région du corps tressé ou tissé et/ou dans lequel la première électrode et la seconde électrode sont couplées électriquement, facultativement dans lequel le dispositif comprend en outre un élément de col sur le corps tressé ou tissé entre une première région du corps tressé ou tissé et la seconde région du corps tressé ou tissé.

16. Système (100) comprenant :
un applicateur électrique selon l'une quelconque des revendications 1 à 15 ; et
un générateur d'impulsions (107) configuré pour générer une pluralité d'impulsions électriques ayant une amplitude d'au moins 0,1 kV et une durée inférieure à 1000 nanosecondes, l'applicateur électrique étant configuré pour se coupler électriquement au générateur d'impulsions.
